# EUROPEAN PATENT APPLICATION

(11) **EP 3 618 071 A1**
(43) Date of publication of application: **04.03.2020**
(21) Application number: 18191148.8
(22) Date of filing: 28.08.2018
(51) Int. Cl.: G16H 10/00

(54) **METHODS FOR SELECTING TUMOR-SPECIFIC NEOANTIGENS**

(71) Applicant: CeCaVa GmbH & Co. KG, 72076 Tübingen (DE)
(72) Inventor: Biskup, Saskia, 72076 Tübingen (DE); Battke, Florian, 72076 Tübingen (DE); Hadaschik, Dirk, 72070 Tübingen (DE); Kyzirakos, Christina, 72070 Tübingen (DE); Kayser, Simone, 72076 Tübingen (DE); Menzel, Moritz, deceased (DE); Armeanu-Ebinger, Sorin, 72076 Tübingen (DE); Feldhahn, Magdalena, 72127 Kusterdingen (DE); Biskup, Dirk, 72076 Tübingen (DE)
(74) Representative: Vossius & Partner Patentanwälte Rechtsanwälte mbB

(57) **Abstract**

Subjects having cancer can be treated in a personalized manner using a patient-specific cocktail of suitable neoantigen or neoepitope peptides and a pharmaceutically acceptable excipient. Selecting suitable neoantigens relies on certain properties of the patient-specific neoantigens predicted or evaluated based on information derived from databases which in turn are derived from prior measurements and observations. Such measurements and observations will be more or less precise and effected to some degree by errors. Thus, the information derived will also be effected by errors to a certain degree. Furthermore, a large number of different properties may need to be taken into account.

In this situation, a selection method is suggested providing for an effective treatment at acceptable costs while reducing the influence of any errors in the underlying databases by binning certain descriptors of neoantigen properties and by improved ranking of the neonantigens according to the binning of the descriptors.

## Description

The present invention relates to the selection of tumor-specific neoantigens of a subject having cancer. The present invention also provides methods using the selected tumor-specific neoantigens in, for example, the treatment or prevention of cancer.

Within the past decade fresh enthusiasm has revived around the possibility of using vaccines as anticancer agents. Data collected by dedicated translational researchers document that a variety of anticancer vaccines, including cell-based, DNA-based, and purified component-based vaccines, are capable of circumventing the poorly immunogenic and highly immunosuppressive nature of tumors and elicit therapeutically relevant immune responses in cancer. Due to observed antitumoral T cell answers induced by tumors, "off-the-shelf" peptide vaccines (targeting mainly unmutated tumor associated antigens like in KRAS, Gastrin G17DT, HSP-CC-96, WT1, VEGF-R and2, hTERT, Her2/neu, KIF20A), recombinant vaccines (MUC-1 and CEA in poxvirus with GM-CSF), live attenuated Listeria Mesothelin-expressing vaccines, irradiated whole allogenic tumor and Listeria and whole inactivated tumor cell vaccines (Algenpantucel-L, Allogeneic GM-CSF) have been evaluated for therapy in cancer.

These studies have generated promising results yet failed in inducing robust, statistically relevant improvement in patient survival. Nevertheless they identified several critical aspects for the design of successful next generation cancer vaccines, namely: cancer vaccines should be tumor specific and distinct from self-proteins, the applied adjuvant should potently activate antigen-presenting cells to stimulate an antigen specific Cytotoxic T lymphocyte (CTL) and T helper lymphocyte mediated immune response and strategies for breaking immunological tolerance and eliciting tumor-associated antigen-specific immunity should be included.

Non-self-antigens like unique neo-antigens created by mutations in a tumor's genome have hitherto been cumbersome to detect. The search including cDNA expression cloning, serologic analysis of recombinant cDNA expression libraries (SEREX), and reverse immunological approaches has become dramatically simplified with the advent of NGS technology. Entire cancer exomes can be sequenced and compared with normal exome, providing the fundamental new opportunity to target the patient individual aberrancy with a vaccine. Such an approach integrates the tremendous heterogeneity of tumors of same tissue type in different individuals and makes an immune response more likely to happen since T cells respond to neo-antigens that have not been subjected to thymic selection processes with a higher affinity. This may explain why driver mutations not necessarily correspond to tumor rejecting antigens underlining that therapeutically useful targets may be generated by individual passenger mutations.

Considerable progress towards significant efficacy has been obtained by combining anticancer vaccines with a relatively varied panel of therapies, which help break the immune suppressive nature of the tumor milieu. These include diverse inhibitors of immune checkpoints, targeted therapies and/or chemotherapeutics (i.e. oxaliplatin) that can provoke immunogenic cell death (ICD).

From WO 2017/205823A1, methods and systems for personalized genetic testing of a subject are known, where a sequencing assay is performed on a biological sample from the subject, which then leads to genetic information related to the subject. It is suggested that nucleic acid molecules are array-synthesized or selected based on the genetic information derived from data of the sequencing assay. At least some of the nucleic acid molecules shall then be used in an assay which may provide additional information on one or more biological samples from the subject or a biological relative of the subject.

However, while genetic information may help in personalizing medical treatment, a large number of problems remain to be solved.

First of all, as with any measurement, the genetic information derived from a person's biological samples may be incorrect to a certain extent, e.g. because the information contains a certain amount of errors. Then, drawing conclusions from genetic information is difficult given that at the time of this invention, medical knowledge still is limited. For example, some rare forms of tumors and cancer may exist that as of yet cannot be attributed with a sufficiently high degree of certainty to specific genetic information. Accordingly, even where a large wealth of genetic data relating to certain diseases exist, for example in the form of libraries, the best information included in such libraries may at one given time be different from the best information included in a similar library at a later time simply because an existing library of genetic data needs to be modified in view of scientific progress.

Then, both any library including medical data and the genetic information obtained from samples of a patient can be rather extensive so that comparing the genetic information obtained from a patient sample to data in one or more libraries can be very computationally intensive.

Also, where it is determined that certain neoantigens might be of particular relevance in view of a cancerous disease a patient suffers from or is believed to suffer from according to the best medical diagnosis available, the selection of neoantigens will depend on which properties the neoantigens have. Such properties might for example be determined in-silico,that is by way of numerical calculation in view of certain assumptions as to their structure. However, neither will the numerical calculations be fully exact nor will the assumptions underlying the calculations or the structure assumed be fully correct.

Nonetheless, despite errors, lack of knowledge, uncertainties and depending on the medical condition of a patients, in certain cases an effective treatment needs to be found both fast and at a cost that is acceptable.

In view of this, there is a need in the art to provide improved methods for ranking personalized neoantigens and uses thereof.

It is thus an object of the invention to inter alia provide novel and inventive methods for ranking personalized neoantigens.

The present invention thus provides a ranking method for ranking neoantigens of a subject having cancer, wherein a plurality of potential neoantigens carrying at least one mutation considered to be cancer-specific is ranked by the steps that
(a) for the subject having cancer a library of potential neoantigens is provided;
(b) for each of a plurality of potential neoantigens from the library, which plurality comprises at least four potential neoantigens, at least two descriptors are determined selected from
   (i) an indicative descriptor indicating whether the neoantigen is known to reside within a cancer-related gene or whether the neoantigen is not known to reside within a cancer-related gene;
   (ii) a classifying descriptor relating to the binning of a value indicative for an allele frequency of the at least one tumor-specific mutation in the neoantigen of the subject into one of at least three different classes ordered according to the intervals of values binned into each class;
   (iii) a classifying descriptor relating to the binning of a value indicative for a relative expression rate of the at least one variant within a neoantigen in one or more cancerous cells of the subject into one of at least three different classes ordered according to the intervals of values binned into each class;
   (iv) a classifying descriptor relating to the binning of a value indicative for a binding affinity of a neoantigen to particular HLA alleles present according to the subject's HLA type, into one of at least three different classes ordered according to the intervals of values binned into each class;
   (v) a classifying descriptor relating to the binning of a value indicative for a relative HLA binding affinity of the subject specific potential neoantigen as compared to the corresponding non-mutated wild-type sequence into one of at least three different classes ordered according to the intervals of values binned into each class;
   (vi) a classifying descriptor relating to the binning of a value indicative for a binding affinity to more than one HLA allele present according to the subject's HLA type, into one of at least three different classes ordered according to the intervals of values binned into each class;
   (vii) a classifying descriptor relating to the binning of a value indicative for the HLA promiscuity of a neoantigen into one of at least three different classes ordered according to the intervals of values binned into each class;
   (viii) a classifying descriptor relating to the binning of a value indicative for the reliability of predicting binding of the subject specific potential neoantigen to a HLA allele of the respective patient into one of at least three different classes ordered according to the intervals of values binned into each class;
   the determination of at least one of the at least two descriptors being such that the number of different classes into which the respective values are binned is smaller than the number of the potential neoantigens of the plurality;
(c) a combined score for each of the plurality of the potential neoantigens is calculated based on the at least two descriptors in a manner weighted such that the maximum possible contribution of at least one descriptor to the combined score will be lower than the maximum possible contribution to the combined score of at least one other descriptor;
(d) a ranking of the plurality of at least four potential neoantigens based on the combined scores is obtained.

The present invention furthermore provides a selection method for cancer-specific neoantigen selection personalized for an individual subject having cancer, wherein from a plurality of potential neoantigens carrying at least one mutation considered to be cancer-specific a selection is made by the steps that for the individual subject having cancer an individual library of potential neoantigens is provided; for each of a plurality of at least four potential neoantigens in the library at least two of an indicative descriptor indicating whether the neoantigen is known to reside within a cancer-related gene or whether the neoantigen is not known to reside within a cancer-related gene; a classifying descriptor relating to the binning of a value indicative for an allele frequency of the at least one tumor-specific mutation in the neoantigen of the subject into one of at least three different classes ordered according to the intervals of values binned into each class; a classifying descriptor relating to the binning of a value indicative for a relative expression rate of the at least one variant within a neoantigen in one or more cancerous cells of the subject into one of at least three different classes ordered according to the intervals of values binned into each class; a classifying descriptor relating to the binning of a value indicative for a binding affinity of a neoantigen to particular HLA alleles present according to the subject's HLA type, into one of at least two different classes ordered according to the intervals of values binned into each class; a classifying descriptor relating to the binning of a value indicative for a relative HLA binding affinity of the subject specific potential neoantigen as compared to the corresponding non-mutated wild-type sequence into one of at least three different classes ordered according to the intervals of values binned into each class; a classifying descriptor relating to the binning of a value indicative for a binding affinity to more than one HLA allele present according to the subject's HLA type, into one of at least three different classes ordered according to the intervals of values binned into each class; a classifying descriptor relating to the binning of a value indicative for the HLA promiscuity of a neoantigen into one of at least three different classes ordered according to the intervals of values binned into each class; a classifying descriptor relating to the binning of a value indicative for the reliability of predicting binding of the subject specific potential neoantigen to a HLA allele of the respective patient into one of at least three different classes ordered according to the intervals of values binned into each class; are determined such that for at least some of the values, the number of different classes, that the classifying descriptor bins the respective values into, is smaller than the number of the potential neoantigens; a combined score for each of the plurality of the potential neoantigens is determined based on the at least two descriptors and in a manner weighted such that the maximum possible contribution of at least one descriptor to the combined score will be lower than the maximum possible contribution to the combined score of at least one other descriptor; and ranking the plurality of at least two potential neoantigens based on the combined scores is determined; and a selection of at least one neoantigen and less than all neoantigens from the plurality of potential neoantigens in response to the ranking is made.

In the above disclosure of a method according to the present invention, reference has been made to the execution of several steps and the derivation and use of certain entities by using expressions such as indicative descriptors, indicative values, classifying descriptors, binning, classes, classes ordered according to the intervals of values, weighting, contributing and so forth. Furthermore, reference will also be made in the following description and appended claims to handicapping , filtering and so forth.

While it is believed that some or most of these common expressions will easily be understood by a person skilled in the art, non-limiting explanations are provided herein below.

In the present invention, reference is made to both an indicative descriptor and to classifying descriptors. The term "descriptor" is used having in mind a standard definition of a so-called molecular descriptor which sometimes is considered a final result of a procedure which transforms chemical information encoded within a symbolic representation of a molecule into a useful number or the result of some standardized experiment. For a specific substance, such a number might e.g. be a binding length within a molecule, a boiling point, the number of carbon atoms and so forth. However, here, when looking at the term "useful number" emphasis in the present application is not on "number" but on "useful".

More precisely, the indicative or classifying descriptors in the present case need not necessarily be a numerical value but could also be e.g. an alphanumerical information.

Regarding the term indicative descriptor indicating whether the neoantigen is known to reside within a cancer-related gene or whether the neoantigen is not known to reside within a cancer-related gene: Frequently, there is knowledge about whether or not a specific neoantigen is known to reside within a cancer-related gene or whether the neoantigen is not known to reside within a cancer-related gene. If, a neoantigen is known to reside within a cancer-related gene, the sentence" Yes, the neoantigen resides within a cancer-related gene", would be an indicative descriptor, whereas a descriptor indicating that neoantigen is not known to reside within a cancer-related gene would be the clear-text sentence "No, the neoantigen is not known to reside within a cancer-related gene" . Obviously, shorter or other desciptors could be used. As nonlimiting examples, the pair "Yes" and "No" would serve the exact same purpose, a pair of "Y" / "N", "Ja"/"Nein", "J"/"N", "Oui"/"Non", "O"/"N" or "A"/"B", a pair of logical flags indicating a logical "0" or "1" asf. Also, instead of an alphanumerical indicative descriptor such as "Y" and "N", numerical values could be used; e.g. a value larger than zero for YES and a value smaller than or equal to zero for NO. While using "0" and "1" would be a standard approach in this case, other values such as ".0543" and "-7.231" could be used as long as they can be clearly distinguished from each other. In particular, a numerical value within a given range of values could be used, for example a value between 0 and 1. This can give additional advantages in certain instances. Suppose the indicative descriptor would be identical to 1 in case the there is a 6 sigma scientific certainty that a given neoantigen is known to reside within a cancer-related gene; while a value of "0,95" shall indicate that only a 5 sigma certainty exists that a given neoantigen is known to reside within a cancer-related gene asf. with a value of 0,5 indicating in this specific case that there currently is no scientific reason at all to assume that a given neoantigen is cancer-related. Here, the indicative descriptor while indicative might also provide additional information.

In the same way, classifying descriptors need not be numerical values either. This can easily be understood as well, and will be explained with respect to the physical size of person as the size is a more commonplace quantity than e.g. a relative HLA binding affinity. Suppose the person is a 6 year old girl that has a physical size of "127cm" corresponding to "4Foot 2 inches" which both are values indicative for the physical size of the person. If the unit used (cm, m, feet) is known, the size can be indicated as "4-2, "1,27", "127", "6-4" asf. Now, to a person not having regular contact with kids, this absolute value will not help to decide whether the girl is rather large for her age or not. However, as the physical size is generally determined and known for a large number of girls, the specific size (127cm) can easily be compared to the size other girls of the same age have. It can thus be established that about 95 % of girls having the same age are smaller. If only three classes are considered, for example small- medium - large, the specific 6 year old girl would most certainly be considered a "large" girl. The classifying descriptor in that case would be "large" but again could also be one of "S", "M","L" or one of "1", "2", or "3" and so forth.

It is important to note that in the example, reference has been made to the size other girls have. In practice it can be determined whether e.g. a specific child is among the smallest 10% of its peer group (peer group= same age, same sex), among the largest 10% of its peer group or somewhere inbetween. (For the sake of completeness: The smallest 10% of 6year old girls have a size up to 110cm; the largest 10% have a size of at least 124cm). Assigning the size of the child to a specific intervall of ranges, (e.g. 0 cm -110 cm; 111 cm -123 cm; >124 cm) is referred to as binning. So, in order to determine that a 6year old girl is a large girl, what is done is that a value indicative for the physical size of the girl is established ("127cm"), the size is roughly compared with other girls by binning ("belongs to the largest 10%") and a classifying descriptor is determined ("this is large girl" or "L" or "3") that relates to the binning of a value indicative for the physical size within a peer group.

Note that in the example the bins or intervalls do not need to have the same size. A girl within the medium range as defined will not differ by more than 12cm from another girl also having medium size. In contrast, a very small girl could be even smaller than 95cm, so the maximum size difference within the "small bin" (or intervall size of the bins) is not the same as in the "medium" bin. It should also be noted that for considering different aspects, different bin sizes can be used. For example, when determining whether a kid should have a somewhat higher or lower chair in school, other limits should be set than when deciding whether in view of a non-average size, medical treatment due to disfunctions is indicated.

Basically, the same holds for quantities other than physical sizes such as a binding affinity of a neoantigen to particular HLA alleles present according to the subject's HLA type; a relative HLA binding affinity of the subject specific potential neoantigen as compared to the corresponding non-mutated wild-type sequence; a relative HLA binding affinity of the subject specific potential neoantigen as compared to the corresponding non-mutated wild-type sequence; the HLA promiscuity of a neoantigen. Here, also, numerical values can be calculated.

The numbers and units to describe such quantities may vary, but it will be obvious to the skilled person how for example, in a manner commonly known, e.g. a binding affinity can be determined. From such standard procedures commonly known, some (numerical) value will be determined e.g. for both the HLA binding affinity of the subject specific potential neoantigen and for HLA binding affinity of the corresponding non-mutated wild-type sequence. Then, when comparing the HLA binding affinity of the subject specific potential neoantigen determined in a manner commonly known to the HLA binding affinity of the corresponding non-mutated wild-type sequence, it could be determined whether the HLA binding affinity of the corresponding non-mutated wild-type sequence wild-type is equal to the HLA binding affinity of the subject specific potential neoantigen or is larger than that or is smaller than that. A corresponding value attributed could e.g. be "+1", "0" or "-1". It will be understood that all binding affinities are positive numbers so when establishing a relation such as "smaller than" or "equal to", a ratio could just as well be determined and it could be checked whether this ratio is larger than 1, smaller than 1 or equal to one. So, a ratio could be determined as such indicative value, a percentage could be determined by multiplying the ratio by 100, a ratio of the squares could be determined as indicative value as indicative value asf.

Regarding classes, several classes or number of binning ranges can be defined. In the example above, the size of a child was stated to be small, medium or large and it has already been stated that different ranges might be useful for different purposes. Also, for some purposes, it might be necessary to establish a different number of classes (such as XS,S,M,L,XL,XXL for absolute sizes when referring to clothing). In the same manner, the number of classes or ranges may differ from 3 for the quantities considered. However, using a number of ranges that is smaller than the number of elements in a sample examined is essential when differences between sample elements are to be disregarded as irrelevant. By using a number of ranges smaller than the number of samples, at least two samples will fall into the same range and hence their absolute difference can be disregarded.

With respect to determining a combined score for each of the plurality of the potential neoantigens based on the at least two descriptors, such combined score of a neoantigen can easily be obtained e.g. by adding certain values; the most simple approach would be to assign each descriptor to a specific numerical value and then add all the values for each neoantigen. (For example, where the descriptor relates to one of the three sizes S M and L, the numbers could be "1", "2" and "3").

However, according to the present invention, the scores are not simply added, but are combined in a specifically weighted manner. Basically, a weighted combination is well known, e.g. from a student of having a main subject of bioinformatics and several subsidiary subjects such as biochemistry. The credit points obtained in different courses usually will be weighted depending on whether or not the course was relating to a subsidiary subject or a main subject of the student, e.g. by multiplying courses in the main subject by a factor of two, that is, by assigning a weight of two. Note that the weights in the present invention are not simply combined in a weighted manner but in a specific manner such that the maximum possible contribution of at least one descriptor to the combined score will be lower than the maximum possible contribution to the combined score of at least one other descriptor- Also, it should be noted that while a simple addition of values certainly is resulting in a combined score, other ways of combining are possible, e.g. adding squared values asf.

It is noted that in the above general description of the invention reference has been made to selecting at least two descriptors from the plurality of descriptors. It will be understood that for each neoantigen that is considered and ranked, the same descriptors are evaluated and used. Furthermore, it is noted that more than two descriptors can be selected. It is also possible that more than three or more than four or more than five descriptors are selected to obtain the ranking from a combined score and again, for all potential neoantigens, the same descriptors will be evaluated and used. Furthermore, it is possible to use all descriptors indicated to obtain a ranking and it would even be possible to use additional, unlisted descriptors that might also contribute in a similar manner to the overall score in a weighted manner to obtain the ranking.

The present inventors have surprisingly and unexpectedly found that the suggested combination of multiple determinations relating to antigen presentation on the surface of tumor cells of a subject in a manner allowing improved selection by a suitable combination of results thus provides patient-individualized tumor vaccines with improved characteristics over the use of prior art prediction methods. This finding is based on the surprising and unexpected results demonstrated in the appended examples. Therein, the effect of personalized neoantigen-based vaccines developed by the methods of the invention is shown (Example 6). Specifically, for a total of 12 patients with various malignancies long-term follow-up data is made available in the appended examples. The data surprisingly and unexpectedly demonstrates that the methods of the present invention can be used to uncover personalized neoantigens resulting in efficient neoantigen-specific T cell immune responses (CD4+ and CD8+).

Accordingly, a clear improvement over existing therapy can be achieved based on peptides selected according to the methods of the present invention. These methods thus provide a surprising and unexpected advantage of resulting vaccines due to the combination of multiple, at least two predictions and determinations and the subsequent combination of results, preferably in a weighted manner.

It has been concluded that surprisingly an improved prediction and selection can be obtained despite the lack of exact knowledge resulting from underlying unprecise or faulty measurements, rounding errors of in-silico calculations asf., if descriptors are binned into one of a few ranges. It is believed that in this way, while the small differences between descriptors will be disregarded most times, their overall value may still be coarsely taken into account without overestimating small, but - given factual precision probably insignificant - differences. For example, it is possible to distinguish values that indicate that the respective descriptor points to a negligible influence, to an influence that albeit small still is considered to be real, or to an influence that is considered to be very large. Specifying one of these classes does not require that the respective value of the descriptor be determined with the highest precision possible. Rather, the errors that the values determined may show will be evened out by the classification. At the same time, by assigning a different weight to the descriptor depending on the range it is classified into, it also is taken into account that a very small value may bear an uncertainty larger than a higher value. Therefore, assigning a particularly low weight or score contribution to an otherwise important factor due to a low value reduces the noise otherwise associated with the low value. It shall be noted that by taking into account a plurality of descriptors, even where the value of one of the descriptor is close to the border of a range, usually minute errors average out.

It should also be noted that even where certain parameters or values are determined in-silico, these determinations may still be dependent on initial physical measurements that as such are error-prone. For example, where a binding affinity is determined, while such determination will depend on assumptions made based e.g. on a molecular structure predicted, the assumptions will still rely on some prior kinetic or other measurement. For example, a binding affinity may be determined based on available data bases that allow calculations based on known properties of certain molecules or functional groups and predicted respective stereochemical structures, but into these data bases data will have been fed from physico-chemical experiments. Thus, in-silico determination of values will not be inherently error-free.

The results achievable demonstrate the superior characteristics of the method used to identify the employed peptides. These methods comprise in a preferred embodiment the combined use of at least several of the following parameters: origin from known cancer-related genes; allele frequency of at least one tumor-specific mutation in the neoantigen of the subject; relative expression rate in a cancerous cell of the subject; binding affinity to particular HLA alleles present according to the subject's HLA type; relative HLA binding affinity of the neoantigen as compared to the corresponding non-mutated wild-type sequence; binding affinity to more than one HLA allele present according to the subject's HLA type, wherein each neoantigen is categorized and each category is given a value, said value can be high if the neoantigen originates from a cancer-related gene; can increase with the allele frequency; can increase with the respective expression rate; can increase with the binding affinity; and can also increase with the relative binding affinity; and can increase with the number of HLA alleles bound. Surprisingly, the combination of the results of at least two of these determinations or parameters, preferably at least three, at least four, at least five or six thereof, results in a ranking of potential neoantigens providing as higher ranked neoantigens peptides, which show a surprisingly increased potential as personalized cancer vaccines. The at least two parameters after categorization are combined, i.e. suitably summed in a weighted manner. Such a weighted approach provides the additional surprising and unexpected effect of an improved ranking with neoantigens being ranked higher that show a very improved potential of being potent cancer vaccines. It was entirely unexpected that a combination could be generalized to the suggested methods as provided herein, which are generally applicable to patients having cancer without the need for individual adaptation. The results of the determination are categorized and then combined in a weighted manner.

In a preferred embodiment of the selection method for cancer-specific neoantigen selection, the combined score for each of the plurality of the potential neoantigens is determined in a manner weighted such that for at least one classifying descriptor, the class dependent contribution to the combined score will for at least one class deviate from a linear relation with class order or will be a penalty.

Using a non-linear relation between class and contribution allows to classify the neoantigen such that an estimated uncertainty of determination can best be taken into account. For example, where a calculated binding affinity is small, rounding errors that cause the same absolute error will result in a large relative change and thus the calculated binding affinity is more affected by errors. Also, where a binding affinity is extremely low, the exact overall value will be of little importance and other factors will become more important. Therefore, it is reasonable to disregard seemingly or actual existing differences and only consider values that are sufficiently large. Accordingly, it is reasonable to choose the range such that in a low range, the contribution to an overall score is small for values within that range. It may also be possible to distinguish the weight of a low affinity that albeit near zero leads to a small but perceptible binding while values of binding affinity that are almost imperceptible and are thus easily outweighed by other factors will contribute significantly less. The number of classes may be larger than three, but using three classes already gives very good results and simplifies a variety of steps in the procedure.

In a preferred embodiment the selection method is executed as a computer-aided selection method wherein at least one of the steps of determining at least one classifying descriptor relating to the binning of a value, determining at least one value subjected to binning to obtain a classifying descriptor, determining a combined score for at least some of the neoantigens, ranking the plurality of at least four potential neoantigens based on the combined scores determined, filtering potential neoantigens, determining the indicative descriptor indicating whether the neoantigen is known to reside within a cancer-related gene or whether the neoantigens is not known to reside within a cancer-related gene, providing an individual library of potential neoantigens in particular in response to at least one of biological sequence data, in particular at least one of DNA sequence data, RNA sequence data, protein sequence data, or peptide sequence data, in particular a combination of such data, and/or data obtained from one of subject specific biological tumor material, such tumor material and additionally subject specific biological non-tumor material, in particular by high-throughput DNA sequencing of at least a number of genes, preferably all genes, high-throughput sequencing of messenger RNA (mRNA) molecules or total RNA, and/or by protein or peptide sequence analysis using tandem mass spectrometry (in particular by proteomics and/or peptidomics) is a step computer aided or implemented.

It should thus be noted that usually at least some, typically most and frequently all steps of the selection and/or ranking method may and shall take place in a computer aided manner. In most cases, implementing such steps in a computer aided manner is far more than a mere convenience. Obtaining results in a sufficiently fast manner usually is vital in the literal meaning of the word as calculating the results without computer support while theoretically feasible would not only be prohibitively expensive but might also lead to a patient having cancer dying before the result is obtained. This holds in particular for in-silico determination of e.g. an allele frequency of the at least one tumor-specific mutation in the neoantigen of the subject, a relative expression rate of the at least one variant within a neoantigen in one or more cancerous cells of the subject, a binding affinity of a neoantigen to particular HLA alleles present according to the subject's HLA type, a relative HLA binding affinity of the subject specific potential neoantigen as compared to the corresponding non-mutated wild-type sequence a binding affinity to more than one HLA allele present according to the subject's HLA type, the HLA promiscuity of a neoantigen, the reliability of predicting binding of the subject specific potential neoantigen to a HLA allele of the respective patient.

Even where it is "only" determined whether a neoantigen is known to reside within a cancer-related gene or whether the neoantigen is not known to reside within a cancer-related gene, the determination will involve a comparison with existing database entries relating to information which genes are known to be cancer related. It should be noted that for such a comparison, even if time needed would be disregarded, use of a computer may be considered vital as well, given that the comparison if done by a human being will be exhausting which in turn leads to errors that might turn out to be fatal even if for no reason other than the fact that a pharmaceutical composition might be produced that due to the errors is not improving the health of the patient. Thus, also in this regard computer-implementation of certain steps should be considered far more than a mere convenience.

In more detail, it is also noted that, within the present invention, it may be determined whether a given neoantigen is known to originate from a cancer-related gene. Cancer-related genes are known to the person skilled in the art from various available data banks including, but not limited to, COSMIC (the Catalogue of Somatic Mutations in Cancer), CCGD (the Candidate Cancer Gene Database), ICGC (International Cancer Genome Consortium),TGDB (the Tumor Gene Database), PMKB (Precision Medicine Knowledgebase), My Cancer Genome or those made available by Galperin et al. (2016) Nucleic Acid Research 45, Issue D1, pp. D1-D11. COSMIC, the Catalogue of Somatic Mutations in Cancer, is a project of the Wellcome Sanger Institute (WSI). WSI is operated by Genome Research Limited (GRL), a charity registered in England with the number 1021457 and a company registered in England with number 2742969, whose registered office is 215 Euston Road, London, NW1 2BE.

CCGD is the Candidate Cancer Gene Database is a product of the Starr Lab at the University of Minnesota (UMN). An in-depth description of this database was published in Nucleic Acids Res. 2015 Jan;43(Database issue):D844-8. doi: 10.1093/nar/gku770. Epub 2014 Sep 4: The Candidate Cancer Gene Database: a database of cancer driver genes from forward genetic screens in mice. ICGC is the International Cancer Genome Consortium, a voluntary scientific organization that provides a forum for collaboration among the world's leading cancer and genomic researchers. The ICGC was launched in 2008 to coordinate large-scale cancer genome studies in tumours from 50 cancer types and/or subtypes that are of main importance across the globe. The ICGC incorporates data from The Cancer Genome Atlas (TCGA) and the Sanger Cancer Genome Project. The consortium's secretariat is at the Ontario Institute for Cancer Research in Toronto, Canada,[3] which will also operate the data coordination center. TGDB (the Tumor Gene Database), is provided by the Baylor College of Medicine, One Baylor Plaza, Houston, Texas. For further details relating to the PMKB (Precision Medicine Knowledgebase), reference is made to J Am Med Inform Assoc. 2017 May 1;24(3):513-519. doi: 10.1093/jamia/ocw148. "The cancer precision medicine knowledge base for structured clinical-grade mutations and interpretations." Huang L1,2, Fernandes H1,3, Zia H1,3, Tavassoli P1,3, Rennert H3, Pisapia D1,3, Imielinski M1,3, Sboner A1,2,3, Rubin MA1,3, Kluk M1,3, Elemento 01,2. Also, it should be noted that a database compilation can be established comprising information from different sources such as several of the above mentioned databases and /or results from own research. In the examples, reference will be found to such a database.

Accordingly, the skilled person is able to determine whether the sequence of a potential neoantigen is located within a known cancer-related gene. A descriptor attributed to the respective neoantigen may change, in particular increase with the probability that a potential neoantigen is located within a known cancer-related gene. In one embodiment, there need only be two discrete values attributed to parameter indicating whether the potential neoantigen originates from a known cancer-related gene or not.

Even the binning and ranking itself may be bothersome if a large number of neoantigens and/or a large number of descriptors are considered. Thus, here, computer-assistance may be preferable as well.

Within the present invention, where the allele frequency of the at least one tumor-specific mutation in the neoantigen in the tumor of the subject is considered, this is based on the assumption that with high allele frequency in the tumor, the neoantigen is more likely to be present and expressed in a high proportion of the tumor cells. Accordingly, the importance and hence overall score contribution attributed to a corresponding parameter increases with the allele frequency in which the tumor-specific mutation is present. In a preferred embodiment of the invention, the corresponding descriptor is chosen according to threshold values determined for high, medium and/or low allele frequency. For example, a high allele frequency may correspond to a value higher or equal to 2/3 times half the tumor content, while a low allele frequency may correspond to a value lower as 1/3 times half the tumor content and values in between may correspond to a medium allele frequency.

Then, it will be noted by a person skilled in the art that filtering out potential neoantigens prior to the selection or handicapping their combined score based on a neoantigen peptide length; a value relating to the neoantigen being a self-peptide or not being a self-peptide; a value relating to the neoantigen expression rate; a value relating to the neoantigen hydrophobicity; and/or a value relating to the neoantigen poly-amino acid stretches may also require lengthy calculations and/or tedious comparison with data base entries. Therefore, here, implementation as a computer aided method step again may be considered at least helpful if not vital as well.

Furthermore, it should be noted that even a computer aided classification, binning and/or determining an overall score from a limited number of neoantigens can be considered vital as implementing these steps as computer aided steps helps to avoid clerical errors.

In a particularly preferred embodiment of the invention, the computer aided steps are executed such that intermediate results obtained can be verified prior to neoantigen selection. Such verification could be executed using an automated expert system although in general it will be preferred to have a human control of the final selection and thus also of at least some of the intermediate results.

In a preferred embodiment of the method the indicative descriptor indicating whether the neoantigen is known to reside within a cancer-related gene or whether the neoantigen is not known to reside within a cancer-related gene is having a first value if the neoantigen is known to be cancer-related and having one of at least two values different from each other and both different from the first value and depending on the likelihood the neoantigen has to be not cancer-related

In other words, it is possible to take into account that a specific neoantigen has only been assumed to be cancer-related even though the assumption has not yet been fully verified with scientific methods to a generally required level of confidence. Such a neoantigen can be distinguished from a neoantigen that has clearly and with high certainty been found to be cancer-related. It can also be distinguished from a neo-antigen that may have been suspected to be cancer-related in the past, but for which sound scientific analysis of a large amount of data has indicated that with a high level of confidence despite an initial assumption to the contrary, such a given other neoantigen is not cancer-related. Thus, for a given neoantigen known to be not cancer-related, the overall score can easily be handicapped by an extremely low or even negative weight or by filtering out the neoantigen entirely from a selection. Also, by assigning a low but positive non-zero weight to a neoantigen that at the time of scoring is considered to be cancer-related even though with a level of confidence still lower than usual due to ongoing scientific evaluations, current best assumptions can be taken into account without overestimating the importance of a given neoantigen. It should be noted that the weight assigned to any given neoantigen in view of its relation to cancer, the descriptor and class and/or the binning intervals may be subject to review by a medical doctor treating a patient and/or a scientific advisor at any time and that over the course of time, inevitably chosen values need be altered as scientific progress is made.

It will thus be understood that the weight of other descriptors and/or the intervals used for their binning may be adapted over time as well.

In a preferred embodiment of the method a step is included of filtering out potential neoantigens prior to selection and/or ranking, or a step of handicapping the combined score of potential neoantigens prior to ranking is included, the handicapping or filtering being in particular based on a value relative to the neoantigen peptide length; a value relating to the neoantigen being a self-peptide or not being a self-peptide; a value relating to the neoantigen expression rate; a value relating to the neoantigen hydrophobicity; and/or a value relating to the neoantigen poly-amino acid stretches.

In this respect, the average skilled person will be aware that according to a present understanding certain neoantigens should not be selected e.g. because the chemical properties thereof are considered to be highly disadvantageous for administering a treatment. In order to prevent that such neoantigens are selected, it is possible to either filter them out before scoring and/or before determining values a descriptor used in scoring is based upon. However, it may be advantageous to include such neoantigens for further considerations rather than filter them out despite certain current concerns. In such a case, the overall score of such neoantigens might be handicapped to an extent sufficient to avoid that they are selected. This may be advantageous in particular as it allows re-evaluation of the overall result should at later times the property of the neoantigen leading to a current handicapping of its score be found to be disregardable in view of further scientific progress.

According to the present understanding, in a preferred embodiment of the invention, the method further comprises a step to ensure that prior to the selection, neoantigens are excluded for which it is likely that a low ranked position will or should be obtained. If such filtering or handicapping is done according to at least one of the parameters peptide length, self-peptides, expression rate, hydrophobicity and/or poly-amino acid stretches, this takes into account that depending on the HLA type, i.e. HLA I or HLA II, to which binding of the neoantigens is restricted, peptide length is known to play an important role. Thus, neoantigens lying outside of lengths of potentially bound peptides by either HLA I or HLA II type proteins can be excluded in a preferred manner. This helps to improve the selection. In a preferred embodiment of the invention, for HLA I restricted peptides, those are excluded that do not comprise between 8 to 11 amino acid residues. For HLA II restricted peptides, it is preferred to exlude those that do not have a length of between 12 and 30 amino acid residues. With respect to self-peptides, it is preferred to exclude those which are known to be part of the endogeneously present sequences. With respect to the expression rate, it is preferred to exclude those neoantigens which are not expressed in the tumor. With respect to hydrophobicity of the neoantigen, it is preferred to exclude those with a high hydrophobicity, whereby high preferably relates to a percentage of more than about 64% hydrophobic amino acids in the potential neoantigen. With respect to poly-amino acid stretches, it is preferred to exclude those which contain three or more identical adjacent amino acid residues.

As can be seen above, binding affinity related values may be considered in selecting neoantigens according to the present invention. In particular, considering the binding affinity to particular HLA alleles, considering the relative HLA binding affinity of the neoantigen compared to a non-mutated wild-type sequence, and considering the binding affinity to more than one HLA allele present according to the subject's HLA type have been mentioned above. However, it will be understood that in certain tumor cells, certain HLA alleles usually present in the patient may not be present. It is advantageous if in such case, such HLA types are excluded from analysis, i.e. binding affinity analysis as defined above, that are not present in the tumor cells.

Therefore, where in a preferred embodiment of the selection method for cancer-specific neoantigen selection at least one of a classifying descriptor relating to the binning of a value of a binding affinity to particular HLA alleles present according to the subject's HLA type, into one of at least three different classes ordered according to the intervals of values binned into each class; a classifying descriptor relating to the binning of a value of a relative HLA binding affinity of the subject specific potential neoantigen as compared to the corresponding non-mutated wild-type sequence into one of at least three different classes ordered according to the intervals of values binned into each class; a classifying descriptor relating to the binning of a value of a binding affinity to more than one HLA allele present according to the subject's HLA type, into one of at least three different classes ordered according to the intervals of values binned into each class; a classifying descriptor relating to the binning of a value of an HLA promiscuity of a neoantigen into one of at least three different classes ordered according to the intervals of values binned into each class; is determined, it is preferred that for determination of the value classified, HLA alleles for which a concentration in tumor cells derived from said subject having cancer lower than normal is assumed are excluded. For the purpose of the present invention, this can be assumed to be the case if the concentration is e.g. 5% lower, or is 10% lower or is 15% lower or is 20% lower or is 25% lower or is 50% lower or is 2/3 lower.

Regarding binding affinity values, according to a preferred embodiment of the present invention, binding affinity related values of the respective neoantigen to particular HLA alleles present according to the subject's HLA type can be determined as part of input data.

It will be understood that scores/binding affinities can be determined by, for example, software tools. It is preferred to use data calculated by software tools such as NetMHC, NetMHCpan and/or SYFPEITHI software. Note that both the NetMHC database and the NetMHCpan database is offered by Technical University of Denmark, DTU Bioinformatics, Kemitorvet, Building 208, DK-2800. SYFPEITHi is a database of MHC ligands and peptide motifs , and has as the correct scientifc citation Hans-Georg Rammensee, Jutta Bach-mann, Niels Nikolaus Emmerich, Oskar Alexander Bachor, Stefan Stevanovic: SYFPEITHI: database for MHC ligands and peptide motifs. Immunogenetics (1999) 50: 213-219 (access via : www.syfpeithi.de).However, any alternative method providing information with respect to the binding affinity of a neoantigen to particular HLA alleles may be used within the present invention. That is, the above exemplified tools may be supplemented and/or replaced with additional/alternative tools. Such tools rely on, for example as SYFPEITHI, a simple model (position specific scoring matrices) based on the observed frequency of an amino acid at a specific position in the peptide sequence to score novel peptides. The training data of SYFPEITHI consist of peptides that are known to be presented on the cell surface. Thus, the training data not only represents the ability of a peptide to bind to a specific MHC allele but also to be produced by the antigen processing pathway (proteasomal cleavage and TAP transport). NetMHC is a neural network-based machine-learning algorithm to predict the binding affinity of peptides to a specific MHC class I allele. The training data consist of experimentally determined binding affinities of peptide:MHC complexes and the sequence of know MHC ligands. NetMHC uses a complex representation of the peptides, based on sequence properties as well as physic-chemical properties of the amino acids. NetMHC can generalize MHC binding of peptides of length 8-11 from training data mostly consisting of peptides of length 9. Thereby it increases the MHC coverage for prediction of peptides of length 9-11 (for many alleles the training data is limited to peptides of length 9). NetMHCpan is a further development of NetMHC. MHC alleles and different peptide lengths are not equally represented in the available training data. NetMHCpan leverages information across MHC binding specificities and peptide lengths and can therefore generate predictions of the affinity of any peptide-MHC class I interaction. Binding prediction is thus available for every known MHC class one allele, and not only for those sufficiently represented in the training data. The above tools are preferably used, however, the skilled person is in a position to adapt these tools to specific needs of the methods provided herein, if required. For example, as an alternative and/or in addition, it would also be possible to determine peptide-HLA I interactions, by e.g. ligandomics (elution of HLA I bound peptides and MS identification) or in vitro binding assays with peptides and HLA I.

Subsequent to determining binding affinities preferably using software tools, in particular one, two, or three of the software tools identified above, the resulting scores of the preferably more than one used software tools may be combined in order to provide a ranking of neoantigens. Obtaining a ranking based on values derived with different tools and/or models reduces errors induced by inter alia the specific model a tools implements. In the invention, this is advantageous as it contributes to obtain a selection even less influenced by errors in initial measurements or imprecise scientific assumptions and estimates.

In a preferred embodiment, threshold values are predetermined in order to provide distinct classes of affinity scores such as high, medium and low affinities for which discrete numerical values are provided.

Within the present invention, a descriptor based on the relative HLA binding affinity of the respective neoantigen as compared to the non-mutated version thereof may be considered. For that purpose, it is preferred to use the same technique as described above. In a preferred embodiment, there are discrete numerical values attributed to neoantigens for which the result lies within predetermined threshold values. For example, a relative binding affinity of the mutated neoantigen as compared with the wildtype version thereof of more than 1.1 may be attributed to a high numerical value (or large contribution to the overall score) whereas a relative binding affinity of below 0.9 may be attributed to a low numerical value (or low contribution to the overall score).

Within the present invention, a descriptor may be based on the number of HLA types for which binding is predicted, i.e. whether binding affinity is predicted for more than one HLA allele whereby the numerical value increases with the number of HLA types bound.

As indicated above, certain HLA alleles should be disregarded in view of a concentration thereof in a tumor cell being lower than normal. In this context, in a preferred embodiment of the selection method for cancer-specific neoantigen selection, HLA alleles are considered to be subject to a HLA haplotype *reduction* derived in view of a tumor transcriptome, a tumor exome or a blood exome or an immunohistochemistry staining of a tumor tissue sample. Thus, genetic data can be used to conclude that a HLA haplotype reduction must be taken into account.

The methods of the present invention may comprise, as a first step, accessing or providing a library of potential neoantigens of a subject having cancer, wherein the neoantigens carry at least one tumor-specific mutation. Thus, as input data, the methods of the present invention may use exome and/or transcriptome sequencing results of the patient having cancer. These sequencing data sets preferably comprise information about somatic missense variants, i.e. non-synonymous single nucleotide variants, the corresponding transcriptome data and the patient's HLA genotype. Based on this information, the methods of the present invention are able to provide a ranking of all potential neoantigens comprised as sequence information in the data sets. The skilled person is well-aware of methods suitable to obtain these data sets from the patient having cancer including sequence information received from tumor cells and healthy cells as a reference. It is preferred to use whole exome sequence data generated by methods well-known in the art.

Once the ranking is done, a selection may take place. In this context, the average skilled person will be aware that it is possible to select more than one neoantigen. In this respect, the selection may comprise one neoantigen or more than one, for example two, three, four, five, six, seven, eight, nine, or ten neoantigens according to their ranked position.

It is useful and preferred to select more than one neoantigen. In case more than one neoantigen is selected, care can be taken to increase the likelihood that the selection is effective by requesting that the neoantigens selected together have certain properties as an ensemble. For example, care can be taken that different HLA types are considered. Even though this may lead to a situation where an ensemble of e.g. six neoantigens is selected that do not constitute the six best scored neoantigens initially considered, the overall selection will still give better results in treating a patient because the likelihood is reduced that all neoantigens will turn out to be ineffective for unknown, unpredicted or underestimated reasons. Also, a possibility exists that an HLA allele is lost in the course of a treatment due to, e.g., immunogenic pressure. For this reason, it is useful to administer further peptides targeting neoantigens which bind to different HLA alleles. Here, targeting a set of neoantigens binding to all available HLA alleles avoids competition for binding to one certain HLA allele and immunodominance effects of one peptide over the others.

In a preferred embodiment of the selection method for cancer-specific neoantigen selection, the method is for selecting for each HLA class I molecule of the patient at least one neoantigen and additionally HLA class II restricted neoantigens.
Such a selection is considered to be advantageous as selecting neoantigens in view of different HLA classes is believed to increase the likelihood that a given selection is effective for treating a patient.

In a preferred embodiment of the selection method for cancer-specific neoantigen selection at least one classifying descriptor is binning the respective value into one of not more than five ordered classes, in particular into not more than four ordered classes, in particular preferably into one of three ordered classes.

Using a large number of ranges that a respective value can be binned into despite being seemingly more precise may not be the most preferred embodiment. On the one hand, the average skilled person will be aware given the present disclosure that a large number of influences need to be factored in. Then, a ranking initially obtained based on an overall score will not determine with absolute certainty that a given neoantigen is selected for a cocktail based on a plurality of cocktails. Accordingly, it may be advantageous to include a given neoantigen in a multi-neoantigen selection only if several factors are also met.

Therefore, although surprising, it has been found sufficient to only distinguish a small number of different ranges. Using a small number of different ranges for any given descriptor not only helps eliminate pseudo-scientific reasonings to rationalize specific thresholds and limits actually set according to personal preferences, but also allows for lower precision of in-silico evaluation of data frequently allowing fewer iterations, calculations with less precision asf. without serious adverse effects. This also helps to reduce the cost of the selection method where particularly lengthy and thus expensive computations should be needed. Therefore, a number of ranges of less than or equal to five is highly preferred. This is even the case where significantly more than four potential neoantigens are ranked, e.g. at least 5, at least 10, at least 15 or at least 20 potential neoantigens are ranked or at least provided from the library prior to filtering. It will be understood that even four ranges usually will suffice, allowing to distinguish a value not discriminable against a zero value, a value not discriminable against a maximum value and two intermediate values. However, in a typical example, it is sufficient and even preferred to have but one intermediate range so that only three ranges "high- medium-low" are needed.

in a preferred embodiment of the selection method for cancer-specific neoantigen selection, all classifying descriptors are binning the respective value into one of not more than five classes, in particular into not more than four classes, in particular preferably into one of three classes. While it is possible to have a different number of possible ranges each descriptor is binned into, a more straightforward and thus faster and cheaper approach is to use the same number of ranges for all classifying descriptors.

It has been found that the number of ranges can be reduced in particular where a sufficiently large number of different descriptors are considered, such as 4, 5, 6 or more descriptors that are all evaluated together. In such a case, there usually will exist more than one pair of descriptors a,b for which the contribution to a combined score S that is determined additively in a manner S=S(a)+S(b) is such that for at least one pair of ranges (a1,a2) of the three, four or more ranges the first descriptor may take and one pair of ranges (b1,b2) the second descriptor may take the contribution S=S(a)+S(b) to the combined score is such that S(a1)+S(b1) > S(a2)+S(b1), S(a2)+S(b1) > S(a2)+S(b2) while S(a1)+S(b2)>S(a2)+S(b1). In other words, a relation may exist such as (S(a1)+S(b1)] > [S(a1)+S(b2)] > [S(a2)+S(b1)] > [S(a2)+S(b2)]. Such property of the influence of descriptors allow to disregard minute differences between certain values as insignificant while still obtaining a very good selection

In a preferred embodiment of the selection method for cancer-specific neoantigen selection the individual library of potential neoantigens is provided in response to exome and/or transcriptome sequencing of subject specific biological material and/or by somatic missense variant identification, in particular of a fresh frozen tumor sample, formalin fixed parrafin embedded tumor material, a stabilized tumor probe, a tumor probe stabilized in PaxGene or Streck Tubes, circulating tumor DNA (ctDNA), or circulating/disseminated tumor cells. PaxGene is a trademark by PreAnalytiX, a joint venture between Becton, Dick-inson and Company and Qiagen, located at Feldbachstrasse, CH 8634 Hombrechtikon. StreckTubes are available from Streck, 7002 S- 109^{th} Street, La Vista, Ne, 68128, United States.

As will be understood by the average skilled person, it is only necessary to provide a sequencing of certain material to obtain data the method can be based upon. It should also be noted that some of the sequencing data can be obtained using material from a patient that may not only be easily obtained but will also be sufficiently stable so as to be transported to a laboratory for sequencing or analysis.

It should be noted and will be understood that it is not necessary to obtain samples, analyze samples, analyze the data obtained by sample analysis, selecting neoantigens and using the selected antigens in preparing a pharmaceutical compositions at one and the same exact location.

Where a plurality of descriptors are evaluated according to the invention, and each may contribute differently according to the respective value the descriptor has for a given neoantigen, the weight assigned to determine the ranking will preferably be such that neoantigens are not simply grouped such that all neoantigens having a first descriptor with a high value are all in one group, all neoantigens having an intermediate value are in a lower ranked group and all neoantigens having a low value are in a third group, and then in each of these groups a second descriptor exists that again splits each (sub) group according to the value this descriptor has asf. until all descriptors are considered. Rather, there usually and preferably will be a situation where the weights each descriptor is assigned in a value-dependent matter is such that a mixing occurs depending on the exact value and the weight assigned. In mathematical terms, thus for at least two descriptors a,b contributing to a combined score S additively in a manner S=S(a)+S(b), at least one pair of values (a1,a2) the first descriptor may take and one pair of values (b1,b2) the second descriptor may take exists such that the contribution S(a)+S(b) to the combined score is such that S(a1)+S(b1) > S(a2)+S(b1), S(a2)+S(b1) > S(a2)+S(b2) while S(a1)+S(b2)>S(a2)+S(b1). In other words, a relation may exist such as [S(a1)+S(b1)] > [S(a1)+S(b2)] > [S(a2)+S(b1)] > [S(a2)+S(b2)].
It is noted that usually a plurality of pairs of descriptors exist that have such a property, in particular at least 2, 3 or 4 pairs and that in a particularly preferred embodiment for at least one descriptor at least two such pairs can be found.

In a preferred embodiment of the selection method for cancer-specific neoantigen selection, this may be achieved inter alia if the maximum possible contribution to the combined score of the descriptor relating to indicating whether or not the neoantigen is known to be cancer-related is larger than the maximum possible contribution to the combined score of any single of the descriptors relating to a relative expression rate in one or more cancerous cells of the subject, a binding affinity to particular HLA alleles present according to the subject's HLA type, a relative HLA binding affinity of the subject specific potential neoantigen as compared to the corresponding non-mutated wild-type sequence, a binding affinity to more than one HLA allele present according to the subject's HLA type, an HLA promiscuity and the reliability of predicting binding of the subject specific potential neoantigen; and/or wherein the maximum possible contribution to the combined score of the descriptor relating to a relative expression rate in one or more cancerous cells of the subject is larger than the maximum possible contribution to the combined score of any single of the descriptors relating to a binding affinity to particular HLA alleles present according to the subject's HLA type, a relative HLA binding affinity of the subject specific potential neoantigen as compared to the corresponding non-mutated wild-type sequence, a binding affinity to more than one HLA allele present according to the subject's HLA type, an HLA promiscuity, and the reliability of predicting binding of the subject specific potential neoantigen; and/or wherein the maximum possible contribution to the combined score of the descriptor relating to a binding affinity to particular HLA alleles present according to the subject's HLA type is larger than the maximum possible contribution to the combined score of any single of the descriptors relating to a relative HLA binding affinity of the subject specific potential neoantigen as compared to the corresponding non-mutated wild-type sequence, a binding affinity to more than one HLA allele present according to the subject's HLA type, an HLA promiscuity, and the reliability of predicting binding of the subject specific potential neoantigen; and/or wherein the maximum possible contribution to the combined score of the descriptor relating to a relative HLA binding affinity of the subject specific potential neoantigen as compared to the corresponding non-mutated wild-type sequence is larger than the maximum possible contribution to the combined score of any single of the descriptors relating to a binding affinity to more than one HLA allele present according to the subject's HLA type, an HLA promiscuity, and the reliability of predicting binding of the subject specific potential neoantigen; and/or wherein the maximum possible contribution to the combined score of the descriptor relating to a binding affinity to more than one HLA allele present according to the subject's HLA type is larger than the maximum possible contribution to the combined score of any single of the descriptors relating to an HLA promiscuity and the reliability of predicting binding of the subject specific potential neoantigen; and/or the maximum possible contribution to the combined score of the descriptor relating to an HLA promiscuity is larger than the maximum possible contribution to the combined score of the descriptors relating to the reliability of predicting binding of the subject specific potential neoantigen. Regarding the reliability of predicting binding, it should be noted that usually binding affinities are numerically calculated using a model and that different models could be used in calculating binding affinities. If more than one model or method of calculation is used, it is likely that the binding affinities calculated with one model will deviate somewhat from binding affinities calculated with another model. Such deviations can be evaluated to determine a reliability of predicting binding, e.g. by considering the absolute or relative difference, the mean variation where a larger number of models are used, and so forth.

It should be noted that in a preferred embodiment of the selection method for cancer-specific neoantigen selection an ensemble consisting of a plurality of different neoantigens is selected. In such a case, the neoantigens of the ensemble can be selected in view of their ranking such that for each of a plurality of the HLA alleles considered the nonfiltered most favorable ranked neoantigen is selected, preferably for each HLA allele the nonfiltered most favorable ranked neoantigen is selected, and such that, if the ensemble comprises more neoantigens than these most favorably ranked neoantigens, then further neoantigens for different alleles are selected starting with HLA-A or B alleles; and preferably further such that if at least two such neoantigens for the same variant, but different alleles starting with HLA-A or B alleles are equally ranked, then a neoantigen with an HLA type hitherto underrepresented in the ensemble is selected, else if at least two such neoantigens for a different variant, but same HLA are equally ranked, then the neoantigen having the higher expression is selected; and preferably further such that both for the case where neoantigens are selected according to their higher expression or the case where a neoantigen with an HLA type hitherto underrepresented in the ensemble is selected, if at least two such neoantigens are equally ranked, then a neoantigen thereof with a higher affinity is selected, preferably a higher affinity according to not the classifying descriptor but according to the original value classified, and preferably further such that if at least two such neoantigens having an equal affinity exist, then the neoantigen having a higher promiscuity is selected and preferably further such that if at least two such neoantigens having an equal affinity exist, then the neoantigen having a lower hydrophobicity is selected.

Thus, it will be noted that there is no guarantee that a neoantigen scoring rather high actually is selected into an ensemble. Rather, the actual selection may depend on properties other high scoring neoantigens have. However, it will be understood that the final process of selecting neoantigens for an ensemble also can be computer implemented and hence automated in particular in view of the additional conditions defined above.

In a preferred embodiment of the selection method for cancer-specific neoantigen selection at least 3 neoantigens are selected. It should be noted that selecting more than one neoantigen is helpful as despite a favorable ranking a situation may occur where other disfavorable factors are not considered at all resulting in a ranking where the highest ranked neoantigen are burdened by such disfavorable factors not considered. The risk of selecting several neoantigens that all are high-ranked but burdened by disfavorable factors however is extremely low. Therefore, selecting at least three neoantigens is preferred and a larger number is even preferred. However, cost may become prohibitive if too large a number of neoantigens is selected. The best number of neoantigens selected may thus not only depend on the specific patient, the progress of his disease and thus the necessity to improve his health faster, but also on the cost of using a large plurality of neoantigens in a pharmaceutical composition rather than using a smaller plurality.

Regarding different contributions of different ranges of different classifying descriptors, it has been found to be preferred for the selection method for cancer-specific neoantigen selection that a classifying descriptor relating to the binning of a value indicative for an allele frequency of the at least one tumor-specific mutation in the neoantigen of the subject into one of at least three different classes ordered according to the intervals of values binned into each class is determined such that a tumor content Y is defined and the value of the allele frequency is defined to be in the highest class if the allele frequency is at least 1/3 of the half tumor content, to be in the lowest class if the allele frequency is no more than 1/6 of half the tumor content Y and else to be in the medium class, and the maximum contribution of the corresponding classifying descriptor if the allele frequency is in the medium class being less than the contribution in case of a highest class and more than the contribution in case of a lowest class. It is noted that while "1/3" and "1/6" are useful limits for the ranges, deviations are possible, e.g. by about 5% or 10% or 15% or 25% of the values indicated. It should be noted that here, reference may be made to either half the tumor content if the somatic mutations in tumor cells are heterozygous or the total tumor content if the somatic mutations are homozygous.

It should be noted that it is possible to re-use respective data and/or intermediate data relating to selection results repeatedly. In particular, it is possible to either re-use the overall selection result repeatedly, for example because a personalized medical treatment is to be effected repeatedly based on the same given selection and/or because the selection result are to be stored together with other patient data as part of a data base that in the end can be used to improve the treatment of the patient or of other patients having a similar diagnosis. It will be understood that a data carrier comprising such a data base will have a significant economical value reflecting the wealth of scientific data included therein and that allowing access to a data base may constitute a source of significant financial income. Access may be provided in an anonymized manner. Providing data in a manner allowing their entry into such a data base is thus considered to be a significant step of both the method of the invention and the production of a data carrier including data relating to a data base that is combining anonymized or non-anonymized patient data and selection related data, in particular binnable values of descriptors usable in the method of selection. Thus, data relating to a selection method for cancer-specific neoantigen selection may be considered a vital and essential part to carry out the method and a vital means to execute the method. It is also possible to store not just the ranking and/or the selected neoantigens but to store intermediate results instead or in addition to the selection. By storing intermediate results such as the values of the descriptors, it becomes possible inter alia to re-classify descriptors to other bins, to change the weight assigned to specific descriptors or to change the number of selected neoantigens. All these measures may help to improve personalized selection methods in the future as scientific progress is made. Therefore, use of the data extends beyond one-time use.

Furthermore, it is obvious that any data obtained is intended to be used to create new products such as personalized pharmaceuticals and /or man- and/or machine-readable prescriptions for such pharmaceuticals. It is envisioned that prescriptions based on the selection may be automatically producible using such data.

It should also be noted that data obtained e.g. by in-silico analysis of genetic data as a step in neoantigen ranking/and or selection of the present invention can be made perceptible by a range of different methods, such as by visualization of data base entries on a monitor or by printing out the results or intermediate. In particular, the limited number of different ranges each descriptor is binned into allows to generate a display where the different range values or score contributions are indicated by different colors. For example, where three different ranges such as high-medium-low are used to bin the value a descriptor may have, it would be possible to assign the colors green, yellow, or red. Then, for a number of neoantigens or for all neoantigens, the weight of a particular descriptor could be used to determine a size of a specifically colored area. For example, where a value of a descriptor is binned into a high range indicating that the neoantigen might be selected in view of this descriptor, the area could be green and if at the same time the descriptor is particularly important such as if the neoantigen is known to be cancer-related, then the green area shown could be made correspondingly large. In this way, a display could be generated where for the respective neoantigens the overall red, yellow and green areas could be shown such that a large green area shows that overall the respective neoantigen should be favored whereas a large red area shows that the respective neoantigen should be disfavored.

It will be obvious that other ways of visualization exist. For example, other colors could be used, the intensity rather than the size of an area could be used to indicate whether or not a neoantigen should be selected, the areas for each descriptor could be shown spaced apart rather than in contact with each other and so forth. However, it will be obvious to the average skilled person that the specific way the computer-implemented method of the invention suggests allows to visualize the intermediate results in a way particularly easy to control. This is an advantage of the present invention as control of intermediate results will not only simplify the implementation of the computer-aided method but will also improve the confidence a user and/or a patient has in the method thus increasing acceptance. Given the above, protection is also sought for a pharmaceutical composition comprising at least one substance determined in response to a result of a selection method as described and disclosed herein. The pharmaceutical composition of the invention may, in one embodiment, be used for treating cancer. In a further embodiment of the invention, the pharmaceutical composition of the invention may be combined with one or more further pharmaceuticals and/or with treatment such as radiation therapy and/or chemotherapy. The skilled person is well-aware of formulations for pharmaceutical compositions and ways how to optimize formulations for therapeutic use. Furthermore, the skilled person is well aware how such pharmaceutical compositions may be administered and how to optimize administration routes for the best therapeutic result. For example, the pharmaceutical composition of the invention may be administered subcutaneously at a site close to the tumour in order to increase the local concentration at the tumor site. The skilled person is also aware of suitable treatment regimens. In this respect, it is preferred that the pharmaceutical composition of the invention is administered continuously, e.g. every four weeks after an initial starting phase with more frequent administration. The skilled person will also be aware of the advantages to be gained by administering on ore more adjuvants together with, or as part of, the pharmaceutical composition.

Furthermore, protection is also sought for using a neoantigen selected in accordance with a method as described and disclosed herein in preparing a personalized pharmaceutical composition.

Then, protection is also sought for a data carrier comprising data relatable to at least one individual patient having cancer, the data carrier carrying data relating to a plurality of potential neoantigens carrying at least one mutation considered to be specific to the cancer of the at least one individual patient in that for each of at least four potential antigens of this plurality of neoantigens at least two of the group (a) thru (h) are provided, with the group (a) thru (h) consisting of (a) an indicative descriptor indicating whether the neoantigen is known to reside within a cancer-related gene or whether the neoantigen is not known to reside within a cancer-related gene and/or a value indicative for a likelihood estimate the neoantigen has to be not cancer-related; (b) a classifying descriptor relating to the binning of a value indicative for an allele frequency of the at least one tumor-specific mutation in the neoantigen of the subject into one of at least two different classes ordered according to the intervals of values binned into each class and/or a value indicative for an allele frequency of the at least one tumor-specific mutation in the neoantigen of the subject into one of at least three different classes, ordered according to the intervals of values binned into each class; (c) a classifying descriptor relating to the binning of a value indicative for a relative expression rate of the at least one variant within a neoantigen in one or more cancerous cells of the subject into one of at least two, preferably at least three different classes ordered according to the intervals of values binned into each class and/or a value indicative for a relative expression rate of the at least one variant within a neoantigen in one or more cancerous cells of the subject; (d) a classifying descriptor relating to the binning of a value indicative for a binding affinity of a neoantigen to particular HLA alleles present according to the subject's HLA type, into one of at least three different classes, ordered according to the intervals of values binned into each class and/or a value indicative for a binding affinity of a neoantigen to particular HLA alleles present according to the subject's HLA type; (e) a classifying descriptor relating to the binning of a value indicative for a relative HLA binding affinity of the subject specific potential neoantigen as compared to the corresponding non-mutated wild-type sequence into one of at least three different classes ordered according to the intervals of values binned into each class and/or a value indicative for a relative HLA binding affinity of the subject specific potential neoantigen as compared to the corresponding non-mutated wild-type sequence; (f) a classifying descriptor relating to the binning of a value indicative for a binding affinity to more than one HLA allele present according to the subject's HLA type, into one of at least three different classes, ordered according to the intervals of values binned into each class and/or a value indicative for a binding affinity to more than one HLA allele present according to the subject's HLA type; (g) a classifying descriptor relating to the binning of a value indicative for the HLA promiscuity of a neoantigen into one of at least three different classes, preferably at least three different classes, ordered according to the intervals of values binned into each class and/or a value indicative for the HLA promiscuity of a neoantigen ; (h) a classifying descriptor relating to the binning of a value indicative for the reliability of predicting binding of the subject specific potential neoantigen to a HLA allele of the respective patient into one of at least three-different classes, preferably at least three different classes, ordered according to the intervals of values binned into each class and/or a value indicative for the reliability of predicting binding of the subject specific potential neoantigen to a HLA allele of the respective patient; and/or the data carrier carrying data relating to neoantigens scoring as obtained by one of the previously claimed methods; and/or the data carrier carrying data relating one or more neoantigens selected according to one of the preceding claims; and/or the data carrier carrying data relating to instructions to produced a pharmaceutical composition comprising at least one substance determined in response to a result of a selection method as described and disclosed herein. The data carrier may comprise an entire data base or part thereof.

Furthermore, protection is sought for a kit comprising at least one of a container for biological material prepared in a manner allowing determination of personalized data usable as input into a ranking and/or selection method as disclosed herein and obtained from a patient having cancer or a data carrier storing personalized (genetic) data usable as individual-related input into a ranking and/or selection method as disclosed herein; the kit also comprising an information carrier carrying information relating to the identification of the patient the kit further comprising instructions to execute a method according to one of the preceding method claims and/or to provide data for the production of a data carrier as described and disclosed herein.

The invention and the method of selecting neoantigens will now be disclosed in more detail.

### Definitions

Unless otherwise defined, understandable and/or obvious from the above, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The term "preferably" is used to describe features or embodiments which are not required in the present invention but may lead to improved technical effects and are thus desirable but not essential.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While aspects of the invention are illustrated and described in detail in the drawings and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below. The invention also covers all further features shown in the figures individually, although they may not have been described in the previous or following description. Also, single alternatives of the embodiments described in the figures and the description and single alternatives of features thereof can be disclaimed from the subject matter of the other aspect of the invention.

### Figure 1: Immune responses toward vaccinated peptides (n=101) in 12 patients.

T cell responses were detected after 12 days *in vitro* stimulation with single peptides, followed by intracellular cytokine staining and FACS analysis to quantify the activation markers IFN-g, TNF, CD154 and CD107a or IL2 in CD4+ and CD8+ T-cells.

### Figure 2: Stimulation index of peptides after 0 and 4 months.

The graph shows that immune responses increased in the course of vaccination (data of one exemplary patient are shown).

**Figure 3****:** For patient No. 2, preexisting CD8⁺ T cell responses were detected against five peptides (here results for one exemplary peptide are shown which were obtained before vaccination started).

Furthermore, in the claims the word "comprising" does not exclude other elements or steps, and the indefinite article "a" or "an" does not exclude a plurality. A single unit may fulfill the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

The following are examples of methods and compositions of the invention. It is understood that various other embodiments may be practiced, given the general description provided above.

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skilled in the art should appreciate, in light of the present disclosure that many changes can be made in the specific embodiments which are disclosed and still obtain a like or similar result without departing from the spirit and scope of the invention. A number of documents including patent applications, manufacturer's manuals and scientific publications are cited herein. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### Example 1 - General method outline

Step 1: Determination of tumor-specific (passenger & driver) mutations by comparison of sequence data from tumor and normal tissue
   - Non-synonymous Single Nucleotide Variants (SNV) and Multiple Nucleotide Variants (MNVs) in close proximity
   - Indels (leading either to a few amino acid changes or to frame shifts and therefore to completely novel amino acid sequences)
   - Fusion genes leading to novel antigens at the breakpoint
Step 2: Definition of mutated peptides based on the mutations found in step 1 and their genomic sequence context.
Step 3: Determination of patient's HLA class I and/or class II status
   - For example, based on the exome data of normal tissue.
Step 4: Identification of mutated peptides that are likely to be presented on the surface of tumor cells based on the list of mutated peptides from step 2 and the HLA status from step 3.
   This can be done for short peptides based on HLA class I status and/or for long peptides based on HLA class II status.
   For example, by prediction of neoantigenic HLA class I restricted epitopes with a length of 8-11 amino acids using the methods SYFPEITHI, netMHC, and netMHCpan and exclusion of self-homologous peptide sequences listed in the UniProtKB/Swiss-Prot Database.
   As algorithms to predict which long peptides (∼17 amino acids) may bind to HLA class II molecules are at present less reliable than those for short class I restricted peptides, class II restricted peptides could be designed manually: 17 nucleotides around the non-synonymous tumor-specific variant are selected such that the variant is in the center position. If variants leading to frameshifts or fusion genes are addressed with long peptides these cover the breakpoints (wt / mutant sequence or DNA locus 1 / DNA locus 2, respectively)
Step 5: Exclusion of mutated peptides which are unlikely to be expressed in the particular tumor entity or the patient's individual tumor. This can, for example, be based on:
   - Tumor specific expression databases
   - Transcriptome analysis allows to control the expression/presence of the variant in the tumor
   - It is envisioned that a proof of existence of peptides on the cancer cell surface might be taken into account if possible.
Step 6: Exclusion of highly hydrophobic peptides to avoid solubility problems during vaccine formulation
   - Exclude peptides with more than 64% hydrophobic amino acids
Step 7: Exclusion of peptides with certain problematic amino acid motives, such as, for example:
   - more than one cysteine (C) which can lead to intra- and inter-molecular disulfide-bridge formation and therefore peptide complexation
   - glutamine (Q) or glutamate (E) at the N-terminus which can spontaneously cyclize to pyroglutamate
Step 8: Determination of loss of HLA alleles in the tumor with respect to the normal tissue tested in step 3. For example, by
   - Determination of HLA class I and/or class II status in the tumor tissue (using tumor exome data or immunohistochemistry)
   - Determination of beta-2 microglobulin status in the tumor tissue (using tumor exome data or immunohistochemistry): if B2M is lost, the HLA class I complex cannot be formed on the tumor cell surface and no class I restricted peptides can be presented on tumor cells.
   - If available, expression of HLA molecules and B2M can be confirmed in the tumor transcriptome data
Step 9: Exclusion of peptides predicted to bind only to HLA molecules which are lost in the tumor (as determined in step 8)
Step 10: Prioritization of mutated peptides to find optimal candidates for vaccination. As an example, a scoring scheme for short HLA class I restricted peptides should take care to
   - Prioritize peptides from known cancer-related genes (CeGaT TUM01, 710 genes)
   - Prioritize variants with high allele frequencies (VAFs) in the tumor. They are more likely expressed by a high proportion of the tumor cells.
   - Prefer variants with a high expression level in the tumor. This can be determined if tumor transcriptome data are available.
   - Prioritize mutated peptides with a stronger predicted HLA binding affinity than the corresponding wildtype peptide
   - Prioritize peptides which are predicted to bind to more than one HLA allele
   - Prioritize peptides which are predicted by more than one algorithm to bind to patient's HLA molecules
Step 11: Combination of peptides into an optimal cocktail as a basis for vaccination
   - Peptides with the highest score are selected in order to cover different variants (driver mutations favored) and if possible all HLA class I and/or class II alleles.
   - The presence of the respective DNA variant can be manually verified in the tumor exome data, in particular with computer support or with orthogonal methods like tumor transcriptome analysis, dPCR or Sanger sequencing.
Step 12: Synthesis of the mutated peptides identified in steps 11
Step 13: Preparation of patient-specific peptide vaccination, for example by
   - Solubilization of single peptides in DMSO
   - Addition of water and pooling of all peptides (final DMSO conc. = 10%; 400 µg each peptide/500 µl injection aliquot). Sterile filtration and filling up of vaccine aliquots in ready-to-use sterile empty glass vials
Step 14: Administration of the patient-specific peptide vaccine

The intradermal injection of the vaccine should be performed together with stimulating adjuvants. MD needs to follow directions of use / application.

### Example 2 - Exemplary method outline for HLA-class I restricted peptides with expression data

### 1. Input

### 1.1. Exome and transcriptome sequencing

- Somatic missense variants from the exome (non-synonymous single nucleotide variants, Indels, gene fusions)
- corresponding transcriptome data,
- Patient's HLA genotype (determined, for instance, from exome data of the patient's blood)

### 1.2. Epitope generation and prediction of binding affinities

- Extraction of 8-11 nucleotides of genomic sequence around a variant position; integration of the variant into the wild-type sequence to generate the neoepitope sequence
- Computation of binding affinity using methods SYFPEITHI, netMHC, netMHCpan

### 2. Filtering

2.1. Filtering of Neoepitopes according to the predicted HLA I binding affinity Exclude neoantigens with affinity>500nM (netMHC/netMHCpan), <50% of max. Score (SYFPEITHI)
2.2. Filtering of self-peptides (UniProtKB/Swiss-Prot HUMAN.fasta)
2.3. Expression data
   - keep if variant allele frequency (VAF) >=5% AND coverage >=20
2.4. Sequence parameters
   - keep if content of hydrophobic AA<=64%
   - If gene is in CeGaT "TUM01" list of known tumor-related genes, keep if number of Cysteines <=1
   - If gene is not in CeGaT "TUM01" list of known tumor-related genes, keep if number of Cysteines =0
   - Keep if poly-amino acid stretches <3 (remove e.g. QQQ)
2.5. HLA haplotype loss
   - HLA typing of tumor transcriptome, tumor exome and blood exome
   - Loss of HLA locus or HLA expression (HLA-A, HLA-B, HLA-C on chr 6, B2M on chr 15) has to be evaluated (CNV calls and allele frequencies in exome sequencing data). If certain HLA haplotype is lost or not expressed, alleles have to be determined and excluded from prediction.

### 3. Scoring

3.1. Cancer-related gene (CeGaT TUM01, 710 genes)
   - Mutations of unknown consequence in in-house TUM01 genes (SCORE 50)
3.2. Allele frequency
   - Define tumor content Y by histopathological evaluation or based on VAFs of detected somatic SNVs
   - High variant allele frequency (VAF): VAF>=2/3*Y/2 (SCORE 45)
   - medium variant allele frequency : 1/3*Y/2<=VAF< 2/3*Y/2 (SCORE 20)
   - low variant allele frequency : 0<VAF<=1/3*Y/2 (SCORE 5)
3.3. Binding affinity
   - The affinity score is calculated on the original scores of NetMHC, NetMHCpan, andSYFPEITHI. The affinity score is calculated for each prediction result as described below and averaged.
   - High affinity (a): a<=50 nm for netMHCpan and netMHC; a>= 75% of max. score for SYFPEITHI (SCORE 40)
   - Medium affinity (a): 50 nM<a<= 200 nM for netMHCpan and netMHC; 60%<=a< 75% of max. Score SYFPEITHI (SCORE 20)
   - Low affinity (a): 200 nM<a<= 500 nM for netMHCpan and netMHC; < 60% of max. score SYFPEITHI (SCORE 10)
3.4. Expression level
   - variant allele frequency in RNA * transcripts per million (RNA VAF * FPKM)
   - Rank according to (RNA VNAF * FPKM). Exclude all with value 0. Count # of remaining variants.
   - Level size (Is) = # of remaining variants / 3
   - High expression range: top ranked variant until top ranked - 1 *Is (SCORE 10)
   - Medium expression range: top ranked - 1 *Is until top ranked - 2*ls (SCORE 5)
   - Low expression range: remaining variants (SCORE 0)
3.5. Binding affinity mutated peptide vs wild-type peptide
   - Calculated on the original scores (NetMHC, NetMHCpan, SYFPEITHI) for the wildtype peptide (WT) and the mutated peptide (MUT). The affinity score is calculated for each prediction result and averaged.
   - Higher: MUT/WT>1,1 (SCORE 10)
   - Equal: 0,9< MUT/WT≤1,1 (SCORE 0)
   - Lower: MUT/WT<0,9 (SCORE -10)
3.6. HLA promiscuity
   - # of different HLA types (HLA) for which binding was predicted
   - High: HLA ≥3 (SCORE 10)
   - Medium: HLA =2 (SCORE 5)
   - Low: HLA =1 (SCORE 0)
3.7. Prediction method congruence
   - # of methods (m) with which binding was predicted
   - High: m=3 (SCORE 5)
   - Medium: m=2 (SCORE 2.5)
   - Low: m=1 (SCORE 0)

### 4. Calculation of Combined Score, Ranking, and Selection

4.1. Compute total score by adding individual scores from previous step.
4.2. Sort peptides according to total score.
4.3. Select top 20 ranked peptides and all peptides that are equally ranked to peptide 20 for each HLA allele and summarize in one list.
4.4. Sort by (in this order): Gene, Total Score, HLA Type
4.5. Mark with Flag 1: Peptide with highest Total Score for each gene. If two peptides for the same gene have equal score, mark both with flag 1
4.6. Sort by (in this order): Flag 1, HLA Type, Total Score
4.7. Mark top 4 peptides in "flag 1" list of each HLA allele with flag 2. If two are equal, mark both with flag 2. If an HLA allele is underrepresented, add best scored peptides from peptides not marked with flag 1. If patient does not have six different HLA alleles, Mark 20/number of HLA alleles per allele (rounded up) with flag 2
4.8. Visually inspect sequencing data for all variants of flag 2 marked peptides
4.9. Select e.g. 7 peptides for synthesis: Best scored peptide for each HLA allele. Fill up with the best scored peptides for different alleles, starting with HLA-A or B alleles.

Two equally ranked peptides for different variant, same HLA:
1. Choose peptide with higher expression
2. Choose peptide with higher affinity (original value)
3. Choose peptide with higher promiscuity
4. Choose peptide with lower hydrophobicity

### Example 3 - Exemplary method outline for HLA-class II restricted peptides without expression data

### 1. Input

1.1. Exome sequencing
   - Somatic missense variants (non-synonymous single nucleotide variants, Indels, gene fusions)
1.2. Epitope generation
   Extraction of 17 nucleotides of genomic sequence around a variant position, with the variant positioned at the center. Generation of the neoepitope by integration of the variant into the wild-type sequence:
   - Missense SNVs: 8+1+8 =17 AA
   - Insertions (of AA size x): 8-(x/2 rounded down) +x+ 8-(x/2 rounded down) = 16 AA if x is equal; =17 AA if x is odd
   - Deletions: 8 AA upstream and 8 AA downstream of deletion; if protein sequence of either site is <8 than add missing AA on the other side so total peptide length is 16 AA
   - Gene fusions: 8 AA upstream and 8 AA downstream of breaking point; if protein sequence of either site is <8 than add missing AA on the other side so total peptide length is 16 AA

### 2. Filtering

2.1. Filtering of self-peptides
2.2. Gene expression estimate Check expression of protein (alternatively RNA) by database search for respective tumor type (Protein atlas, if not available, GEO). Exclude peptides of genes that are not expressed in tumor type.
2.3. Sequence parameters
   - keep if % hydrophobic AA<=64
   - If gene is in CeGaT "TUM01" list of known tumor genes, keep if number of Cysteines <=1
   - If gene is not in CeGaT "TUM01" list of known tumor genes, keep if number of Cysteines =0
   - Keep if poly-amino acid stretches <3 (remove e.g. QQQ)

### 3. Scoring

3.1. Cancer gene (CeGaT TUM01, 649 genes)
   - Mutations of unknown consequence listed in CeGaT TUM01 (SCORE 50)
3.2. Allele frequency
   - Define tumor content Y
   - High variant allele frequency (VAF): VAF>=2/3*Y/2 (SCORE 45)
   - medium variant allele frequency: 1/3*Y/2<=VAF< 2/3*Y/2 (SCORE 20)
   - low variant allele frequency: 0<VAF<=1/3*Y/2 (SCORE 5)
3.3. Gene expression estimate
   Check expression of protein by database search for respective tumor type (Protein atlas, if not available, GEO). Mark expression level in respective tumor tissue: high/medium/low/heterogenic. "High" is assigned SCORE 10, "Medium" is assigned SCORE 5, "Low" is assigned SCORE 0.
3.4. If HLA class I peptides were already selected for the patient (see example 2), exclude all HLA class II peptides already covered by class I peptides.

### 4. Calculation of Combined Score, Ranking, and Selection

4.1. Compute total score by adding individual scores from previous step
4.2. Sort peptides according to total score
4.3. Select top 3 peptides.
   Given two equally ranked peptides for different variants:
   1. Choose peptide with higher expression
   2. Choose peptide with higher VAF
   3. Choose peptide with lower hydrophobicity

### Example 4 : Comparison of peptide ensembles obtained according to different methods

As stated above, for treating a patient, it is typically useful and preferred to select more than one neoantigen. In case more than one neoantigen is selected, care can be taken to increase the likelihood that the selection is effective by requesting that the neoantigens selected together have certain properties as an ensemble. For example, care can be taken that different HLA types are considered.

However, when selecting a plurality of neoantigens such that the ensemble together has certain properties, care must be taken that the overall ensemble still has favorable properties. It will be understood that comparing the results obtained by different selection methods in a statistically relevant and thus very large number of patients is not an option ethically defensible. Therefore, the results obtained by different methods must be compared in a different manner.

To this end, based on data obtained from an actual patient an ensemble of 5 peptides was determined and the results thereof evaluated in view of averages of values of the ensemble. In particular, for each of the respective 5 peptides obtained by the different methods, allele frequency, a degree of promiscuity, binding affinity and difference between wildtype peptide and mutated peptide were compiled. Furthermore it was indicated what gene the peptide belongs to, whether the gene was known to be cancer-related, and also the HLA allele was determined.

This compilation is then used to compare the quality of the different ensembles obtained.

### a - Ensemble by Random Selection

In a first approach, five peptides were randomly selected from a list of peptides predicted to be neoantigens for a tumor.

For these 5 peptides, allele frequency, promiscuity, binding affinity and difference between wildtype peptide and mutated peptide were calculated. Furthermore, it was determined what gene the peptide belongs to, whether the gene was known to be cancer-related, and the HLA allele was determined.

The following results were obtained:

| Peptide | Gene | VAF | Tumor gene | Affinity | Diff. Affnty W/M | Promisc | Peptide | HLA allele |
|---|---|---|---|---|---|---|---|---|
| 1 | CNN2 | 0,068 | no | 64 | 5 | 1 | DPGEAPEY | HLA-B*35:01 |
| 2 | SFI1 | 0,052 | no | 177 | -213 | 1 | QLLYVQKGKQK | HLA-A*03:01 |
| 3 | TRAPPC8 | 0,054 | no | 175 | -391 | 1 | FTSRSLNV | HLA-C*05:01 |
| 4 | LONP1 | 0,125 | no | 138 | -91 | 1 | GFTLFVETSLR | HLA-A*31:01 |
| 5 | ALAS1 | 0,102 | no | 213 | -170 | 2 | RSDPSFPK | HLA-A*03:01 |

It was thus found that the mean allele frequency of the five peptides is rather low, having a value of about 8%. The mean binding affinity is 153, the mean difference between wildtype binding affinity and mutant binding affinity is a mere -172. The ensemble covers four different HLA alleles but none of the peptides bind to more than one HLA allele and none relates to a tumor gene.

### b - Ensemble according to score of unweighted parameters

While a random selection of peptides is an extremely easy approach, it will be obvious to a skilled person that a variety of parameters may be considered to improve the selection. Accordingly, the random selection given above basically can serve as a base line.

If some general knowledge of topics such as tumor genetics, depletion of proteins in a cell, and the presentation of peptides at the cell surface is used, a number of parameters can be selected for establishing a score of peptides. Using such a score, five peptides can be selected that each relate to a different gene.

For this example, it is considered whether the neoantigen is known to reside within a cancer-related gene.
Then, an average skilled person might want to consider whether the difference between the HLA binding affinity of the (subject specific) potential neoantigen and the corresponding non-mutated wild-type is large or not; in other words, the relative HLA binding affinity of the potential neoantigen as compared to the corresponding non-mutated wild-type sequence may be considered.
Also the binding affinity of the mutated peptide may be considered as obtained, using the values obtained both by NetMHC and NetMHCpan and averaging these values.
Finally, the promiscuity is taken into account,i.e. the number of alleles a peptide can bind to.

In order to select five peptides based on these four parameters, an overall score must be determined. Here, it must be taken into account that the different parameters will have very different values. In order to determine an overall score, a simple approach is to rank the set of peptides with respect to each parameter, giving four rankings for each peptide considered and to then add all the rankings a peptide has obtained. An overall "score" is determined based on this sum, favoring those peptides having the lowest rank.

Using this sum, a selection of five peptides can then be made, taking care that any gene is selected only once. Accordingly, a peptide will be selected for the ensemble only if all higher ranked peptides selected relate to a different gene.

The following results were obtained:

| Peptide | Gene | VAF | Tumor gene | Affinity | Diff. Affnty W/M | Promisc | Peptide | HLA allele |
|---|---|---|---|---|---|---|---|---|
| 1 | LONP1 | 0,125 | no | 134 | -7442 | 1 | LAWTAMGGF | HLA-B*35:01 |
| 2 | MED16 | 0,255 | no | 70 | -10565 | 1 | SPGDRLTEIY | HLA-B*35:01 |
| 3 | GBP4 | 0,109 | no | 56 | -17150 | 2 | RSFQEYMAQMK | HLA-A*03:01 |
| 4 | PRR21 | 0,282 | no | 28 | 19 | 1 | SSTPLHPR | HLA-A*31:01 |
| 5 | PERM1 | 0,320 | no | 14 | 4 | 1 | RYFRRQAGQGR | HLA-A*31:01 |

It was thus found that for the five peptides suggested, a very high affinity with a mean value of 60 was achieved and that the mean difference between wildtype binding affinity and mutant binding affinity is -7026. The mean allele frequency of the five peptides is about 22%. No tumor genes have been selected.

### c - Ensemble according to score of parameters weighted according to the invention

While the approach under "b" is an improvement over a random selection, it will be understood that selecting peptides relating to tumor genes might improve the overall results. To evaluate whether this leads to any improvement, a method similar to "b" is executed, with the only difference that once the sum of the four rankings is obtained, first of all, peptides relating to tumor genes are selected. Only in case no further tumor gene related peptides are found may high ranking non-tumor gene related peptides be selected,

In this manner, the following selection has been made:

| Peptide | Gene | VAF | Tumor gene | Affinity | Diff. Affnty W/M | Promisc | Peptide | HLA allele |
|---|---|---|---|---|---|---|---|---|
| 1 | CHD4 | 0,109 | yes | 122 | -30863 | 1 | WMDLKKCR | HLA-A*31:01 |
| 2 | PIK3CA | 0,112 | yes | 111 | -12291 | 1 | YFMKQMNDAR | HLA-A*31:01 |
| 3 | PARK2 | 0,065 | yes | 56 | -28 | 1 | RNDWTVQNF | HLA-C*04:01 |
| 4 | LONP1 | 0,125 | no | 134 | -3119 | 1 | LAWTAMGGF | HLA-B*35:01 |
| 5 | MED16 | 0,255 | no | 70 | -9466 | 1 | SPGDRLTEIY | HLA-B*35:01 |

As can be seen, the five peptides suggested have a mean affinity value of 71, which is slightly higher than that obtained in method "b" and a larger difference of wild type and mutant binding affinities, the mean difference being -11358. The mean allele frequency is 13% and of the five peptides selected, three relate to tumor genes.

### d - Ensemble selection according to invention

Considering that a selection based primarily on tumor genes may result in selection of peptides for an ensemble that might have a variety of disadvantageous properties, a scoring according to the invention is suggested such that inter alia, the overall score a peptide may obtain will not be solely dominated by whether or not the peptide is tumor gene related.

In this manner, it can e.g. be avoided that tumor gene related peptides having hardly usable binding affinities will be preferred over non-tumor gene related peptides.

The following results were obtained:

| Peptide | Gene | VAF | Tumor gene | Affinity | Diff. Affnty W/M | Promisc | Peptide | HLA allele |
|---|---|---|---|---|---|---|---|---|
| 1 | CHD4 | 0,109 | yes | 122 | -30863 | 1 | WMDLKKCR | HLA-A*31:01 |
| 2 | PIK3CA | 0,112 | yes | 129 | -16807 | 1 | FMKQMNDAR | HLA-A*31:01 |
| 3 | GBP4 | 0,109 | no | 56 | -17150 | 2 | RSFQEYMAQMK | HLA-A*03:01 |
| 4 | PARK2 | 0,065 | yes | 56 | -28 | 1 | RNDWTVQNF | HLA-C*04:01 |
| 5 | PERM1 | 0,320 | no | 14 | 4 | 1 | RYFRRQAGQGR | HLA-A*31:01 |

In the example given, it can be seen that non-tumor gene peptide in GBP4 has a better score than the lower ranked tumor-gene related peptide in PARK2. Furthermore, a peptide having a promiscuity of 2 suggested according to method "b" but disregarded using method "c" is included in the ensemble.

The preferred method suggests five peptides having a mean affinity similar to method "c" (with a mean value of 75), but showing a larger difference of wild type and mutant binding affinities, the mean difference being -12969. The average allele frequency is 14% and thus higher than in method "c". As in method "c" three out of five peptides relate to tumor genes.

This shows that the method according to the invention using an improved score is giving results that improve on allele frequency and difference of wild type and mutant binding affinities while not affecting affinity itself.

The following comparison summarizes these findings indicating that for an overall ensemble obtained according to the method of the present invention, relevant properties are on average found to be very good. It can be appreciated that administering these peptides in a pharmaceutical composition will give very good results in treating a patient because the likelihood is reduced that all neoantigens will turn out to be ineffective for unknown, unpredicted or underestimated reasons. Also when a HLA allele is lost in the course of the treatment due to immunogenic pressure, the preferred ensemble will contain further peptides targeting neoantigens which bind to different HLA alleles. Here, targeting a set of neoantigens binding to several HLA alleles reduces the impact of competition for binding to one certain HLA allele and immunodominance effects of one peptide over the others.

| **Method** | **Avg VAF** | **Affinity** | Diff. Affnty W/M | **Tumor gene** | **Promis** | **#Alleles covered** |
|---|---|---|---|---|---|---|
| **Random** | 0,0802 | 153,42 | -172,04 | 0 | 1,2 | 4 |
| **N1** | 0,2182 | 60,15 | -7.026,75 | 0 | 1,2 | 3 |
| **N2** | 0,1332 | 71,11 | -11.358,10 | 3 | 1,0 | 3 |
| **Invention** | 0,1430 | 75,11 | -12.968,74 | 3 | 1,2 | 3 |

### Example 5 - Vaccination regime of adult patients

| | |
|---|---|
| **Vaccine:** | Intra-dermal injections of formulated peptides (400 µg each/dose); short class I restricted peptides (8-11 amino acids) & long class II restricted peptides (∼17 amino acids). Note that 400 µg were used independent of the weight of a patient. |
| | |
| **Adjuvants:** | Subcutaneous injection of Leukine (GM-CSF) |
| **Administration:** | Day 1, 3, 8, 15, 29. Monthly repeats. |

### Example 6 - Personalized neoantigen-targeting vaccines

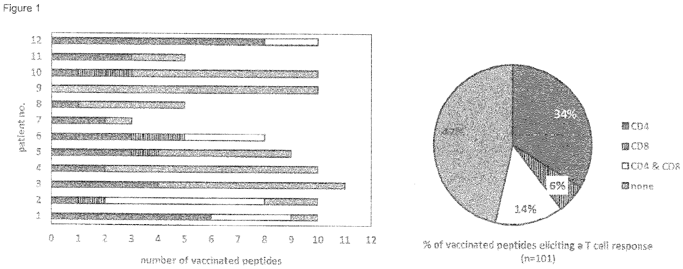

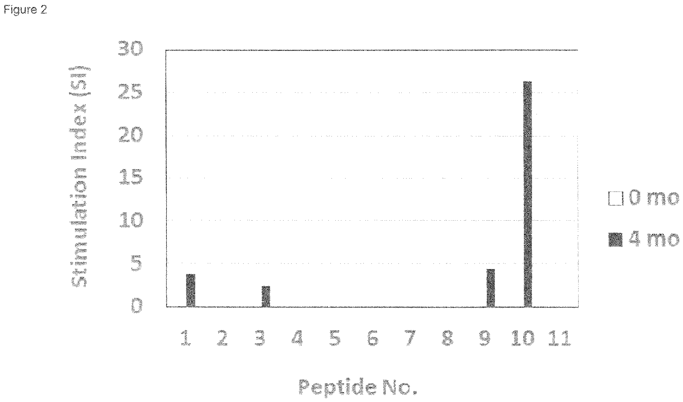

The methods described above have been used to develop personalized neoantigen-based vaccines for the treatment of cancer patients. Each resulting vaccine consisted of up to 20 peptides resembling distinct non-self antigens derived from tumor-specific mutations (neoantigens), not present in the normal tissues of the respective patient. In order to elicit a sustained immune response against cancer cells presenting such neoantigens via MHC on their surface, a peptide vaccine was repeatedly applied together with an immunostimulatory adjuvant (Leukine, GM-CSF). According to the in-house established vaccination schedule, the personalized peptide vaccine was injected intradermally in the upper thigh or abdomen on days 1, 3, 8, 5 29 and subsequently every 4 weeks (0.4 mg each peptide/injection). In order to increase the immune response to the vaccinated peptides, the adjuvant Leukine (GM-CSF) was additionally injected subcutaneously in close proximity to the vaccination site (83 µg/injection).
Each vaccination cocktail consisted of short peptides (8 to 11 amino acids) and long peptides (15 to 21 amino acids). While short peptides are taken up and presented by antigen presenting cells (APCs) via MHC I molecules in order to activate neoantigen-specific cytotoxic T cells (CD8+), long peptides are internalized, processed and presented by APCs via MHC II molecules in order to activate neoantigen-specific T-helper cells (CD4+). The aim was to activate both T-cell populations, as they are thought to play distinct but complementary roles in the fight against tumor cells (Braumuller, H.; Wieder, T.; Brenner, E.; Assmann, S.; Hahn, M.; Alkhaled, M. et al. (2013) T-helper-1-cell cytokines drive cancer into senescence in: Nature 494 (7437), S. 361-365. DOI: 10.1038/nature11824; Dudley, M. E.; Gross, C. A.; Langhan, M. M.; Garcia; Sherry, R. M.; Yang, J. C. et al. (2010): CD8+ enriched "young" tumor infiltrating lymphocytes can mediate regression of metastatic melanoma in: Clinical cancer research : an official journal of the American Association for Cancer Research 16 (24), S. 6122-6131. DOI: 10.1158/1078-0432.CCR-10-1297; Heemskerk, B.; Kvistborg, P.; Schumacher, T. N. (2013): The cancer antigenome in : The EMBO journal 32 (2), S. 194-203. DOI: 10.1038/emboj.2012.333; Kreiter, S.; Vormehr, M.; van de Roemer, N.; Diken, M.; Lower, M.; Diekmann, J. et al. (2015): Mutant MHC class II epitopes drive therapeutic immune responses to cancer in: Nature 520 (7549), S. 692-696. DOI: 10.1038/nature14426; Schumacher, T. N.; Schreiber, R. D. (2015): Neoantigens in cancer immunotherapy in Science (New York, N.Y.) 348 (6230), S. 69-74. DOI: 10.1126/science.aaa4971; Tran, E.; Turcotte, S.; Gros, A.; Robbins, P. F.; Lu, Y. C.; Dudley, M. E. et al. (2014): Cancer immunotherapy based on mutation-specific CD4+ T cells in a patient with epithelial cancer in: Science (New York, N.Y.) 344 (6184), S. 641-645. DOI: 10.1126/science.1251102.).
A number of patients suffering from tumors of diverse origin and late stage, which were refractory to standard therapies, were treated on a compassionate-use basis with personalized neoantigen-targeting multi-peptide vaccines designed by the methods described in the invention. The use of the personalized vaccines was registered by the local authorities in Germany (Regierungsprasidium Tuebingen) and all German regulations for compassionate use treatment were followed. In general the patients showed promising outcomes. The first patient, suffering from a pancreatic carcinoma, started with vaccinations 4.5 years ago and is still alive (Sonntag K., Hashimoto H., Eyrich M., Menzel M., Schubach M., Docker D., Battke F., Courage C., Lambertz H., Handgretinger R., Biskup S., Schiibach K. Immune monitoring and TCR sequencing of CD4 T cells in a long term responsive patient with metastasized pancreatic ductal carcinoma treated with individualized, neoepitope-derived multipeptide vaccines: a case report in J Transl Med. 2018 Feb 6;16(1):23. DOI: 10.1186/s12967-018-1382-1). For a total of 12 patients with various malignancies long-term follow-up data including immunogenicity data are shown in Figure 1. Each patient received repeated vaccinations utilizing between 3 and 11 peptides for at least 2.5 months before vaccine specific T-cell responses were assessed by intracellular cytokine staining and FACS analysis. Vaccine-specific T-cell responses were detected in all of these patients, except for one (patient no 9). An immune response was detectable to 53% of vaccinated peptides (54/101). Several peptides elicited CD4+, as well as CD8+ T cell responses (14%). Overall, 48% of the vaccinated peptides were recognized by CD4+ and 20% by CD8+ T cells. For nine patients, evaluable data from several subsequent time points were available, and for seven of those, immune responses increased in the course of the vaccination schedule (exemplified in Fig. 2).

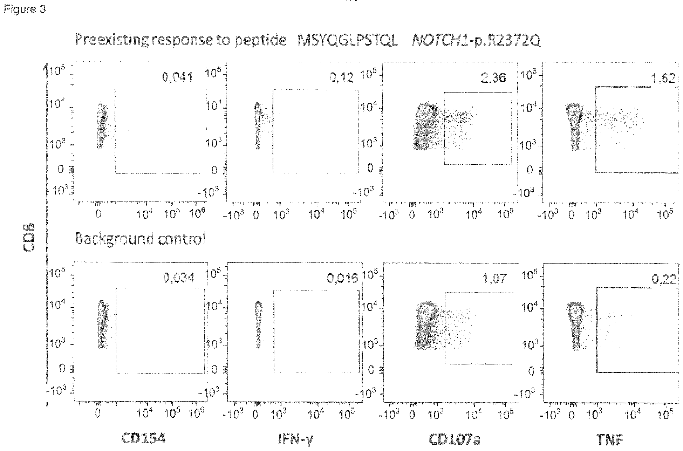

Prior to vaccination one breast cancer patient (No. 2), displayed already existing CD8+ immune responses against five of 10 peptides included in the vaccination cocktail. Therefore, the in-silico predicted neoantigen-peptides of the vaccine must have been presented via MHC molecules on tumor cells in vivo and prior to vaccination. This, in turn, led to a naturally occurring and efficient priming of neoantigen-specific T cells (Fig. 3: exemplary immune response to peptide MSYQGLPSTQL, NOTCH1-p.R2372Q). These results highlight that indeed the selected neoantigens were presented on the tumor-cell surface and that the applied neoantigen prediction and selection procedure is capable of identifying such novel and immunogenic tumor-epitopes. As the described patient is currently in complete remission, it is tempting to speculate that the tumor-specific immune response may have contributed to the positive outcome. Furthermore, these findings affirm the conclusion that the induction of a neoantigen-specific immunity in patients, who have not established a natural immune response against the same tumor-antigens before, might be of high clinical relevance.

In summary, results from immune-monitoring experiments performed for 12 vaccinated cancer patients demonstrated that efficient neoantigen-specific T cell responses (CD4+ and CD8+) are elicited upon vaccine injection. Such immune responses were observed to continually increase during the treatment. Preexisting immune responses against vaccine peptides which were detected prior to the vaccination further indicated, that the respective neoantigens were presented to the immune cells on the tumor cell surface before vaccination and that the established neoantigen selection process of the invention leads to the efficient selection of such immunogenic tumor-specific epitopes.

From the above, it is obvious that the disclosure of the present invention also comprises inter alia a pharmaceutical composition prepared as suggested in either the claims and/or the description for use in treating cancer. What is also disclosed is the use of a neoantigen selected in accordance with a method according to any of the claims in preparing a personalized pharmaceutical composition. Furthermore, a method of treating cancer, comprising administering to a patient in need thereof an effective amount of a pharmaceutical composition as claimed is suggested.

## Claims

1. A ranking method for ranking neoantigens of a subject having cancer, wherein a plurality of potential neoantigens carrying at least one mutation considered to be cancer-specific is ranked by the steps that
(a) for the subject having cancer a library of potential neoantigens is provided;
(b) for each of a plurality of potential neoantigens from the library, which plurality comprises at least four potential neoantigens, at least two descriptors are determined selected from
(i) an indicative descriptor indicating whether the neoantigen is known to reside within a cancer-related gene or whether the neoantigen is not known to reside within a cancer-related gene;
(ii) a classifying descriptor relating to the binning of a value indicative for an allele frequency of the at least one tumor-specific mutation in the neoantigen of the subject into one of at least three different classes ordered according to the intervals of values binned into each class;
(iii) a classifying descriptor relating to the binning of a value indicative for a relative expression rate of the at least one variant within a neoantigen in one or more cancerous cells of the subject into one of at least three different classes ordered according to the intervals of values binned into each class;
(iv) a classifying descriptor relating to the binning of a value indicative for a binding affinity of a neoantigen to particular HLA alleles present according to the subject's HLA type, into one of at least three different classes ordered according to the intervals of values binned into each class;
(v) a classifying descriptor relating to the binning of a value indicative for a relative HLA binding affinity of the subject specific potential neoantigen as compared to the corresponding non-mutated wild-type sequence into one of at least three different classes ordered according to the intervals of values binned into each class;
(vi) a classifying descriptor relating to the binning of a value indicative for a binding affinity to more than one HLA allele present according to the subject's HLA type, into one of at least three different classes ordered according to the intervals of values binned into each class;
(vii) a classifying descriptor relating to the binning of a value indicative for the HLA promiscuity of a neoantigen into one of at least three different classes ordered according to the intervals of values binned into each class;
(viii) a classifying descriptor relating to the binning of a value indicative for the reliability of predicting binding of the subject specific potential neoantigen to a HLA allele of the respective patient into one of at least three different classes ordered according to the intervals of values binned into each class;
the determination of at least one of the at least two descriptors being such that the number of different classes into which the respective values are binned is smaller than the number of the potential neoantigens of the plurality;
(c) a combined score for each of the plurality of the potential neoantigens is calculated based on the at least two descriptors in a manner weighted such that the maximum possible contribution of at least one descriptor to the combined score will be lower than the maximum possible contribution to the combined score of at least one other descriptor;
(d) a ranking of the plurality of at least four potential neoantigens based on the combined scores is obtained.

2. A method according to claim 1,
wherein the combined score for each of the plurality of the potential neoantigens is calculated in a manner weighted such that for at least one classifying descriptor, the class dependent contribution to the combined score will for at least one class deviate from a linear relation with class order or will be a penalty.

3. The method according to any of the previous claims, wherein for at least two descriptors (a,b) contributing to a combined score S additively in a manner S=S(a)+S(b), at least one pair of values (a1,a2) the first descriptor may take and one pair of values (b1,b2) the second descriptor may take exists such that the contribution S(a)+S(b) to the combined score is such that S(a1)+S(b1) > S(a2)+S(b1),S(a2)+S(b1) > S(a2)+S(b2) but S(a1)+S(b2) > S(a2)+S(b1).

4. A method according to any of the previous claims,
wherein the individual library of potential neoantigens is provided in response to exome and/or transcriptome sequencing of subject specific biological material and/or by somatic missensevariant identification, in particular using at least one of a fresh frozen tumor sample, formalin fixed parrafin embedded tumor material, a stabilized tumor probe, a tumor probe stabilized in PaxGeneTubes, ctDNA, or circulating/disseminated tumor cells;
and/or wherein the indicative descriptor indicating whether the neoantigen is known to reside within a cancer-related gene or whether the neoantigens is not known to reside within a cancer-related gene is having a first value if the neoantigen is known to be cancer-related and having one of at least two values different from each other and both different from the first value and depending on the likelihood the neoantigen has to be not cancer-related;
and/or wherein a step is included of filtering out potential neoantigens prior to a subsequent selection, or of handicapping the combined scored of potential neoantigens prior to ranking,
the handicapping or filtering being in particular based on at least one of
a value relating to the neoantigen peptide length;
a value relating to the neoantigen being a self-peptide or not being a self-peptide;
a value relating to the neoantigen expression rate;
a value relating to the neoantigen hydrophobicity; and/or
a value relating to the neoantigen poly-amino acid stretches.

5. A computer-aided method according to one of the previous claims wherein at least one of the steps of
- determining at least one classifying descriptor relating to the binning of a value,
- determining at least one value subjected to binning to obtain a classifying descriptor,
- calculating a combined score for at least some of the neoantigens,
- ranking the plurality of at least four potential neoantigens based on the combined scores determined,
- filtering out potential neoantigens,
- determining the indicative descriptor indicating whether the neoantigen is known to reside within a cancer-related gene or whether the neoantigens is not known to reside within a cancer-related gene,
- providing an individual library of potential neoantigens in particular in response to at least one of biological sequence data, in particular at least one of DNA sequence data, RNA sequence data, protein sequence data, or peptide sequence data, in particular a combination of such data, and/or data obtained from one of subject specific biological tumor material such tumor material and additionally subject specific biological non-tumor material, in particular by high-throughput DNA sequencing of at least a number of genes, preferably all genes, high-throughput sequencing of messenger RNA (mRNA) molecules or total RNA, and/or by protein or peptide sequence analysis using tandem mass spectrometry, in particular by proteomics and/or peptidomics,
is a step computer aided or implemented.

6. A method according to one of the previous claims, wherein
at least
one of a classifying descriptor relating to the binning of a value of a binding affinity to particular HLA alleles present according to the subject's HLA type, into one of at least three different classes ordered according to the intervals of values binned into each class;
a classifying descriptor relating to the binning of a value of a relative HLA binding affinity of the subject specific potential neoantigen as compared to the corresponding non-mutated wild-type sequence into one of at least three different classes ordered according to the intervals of values binned into each class;
a classifying descriptor relating to the binning of a value of a binding affinity to more than one HLA allele present according to the subject's HLA type, into one of at least three different classes ordered according to the intervals of values binned into each class;
a classifying descriptor relating to the binning of a value of an HLA promiscuity of a neoantigen into one of at least three different classes ordered according to the intervals of values binned into each class;
is determined and wherein for determination of the value classified, HLA alleles for which a concentration in tumor cells derived from said subject having cancer lower than normal is assumed are excluded.

7. The method according to one of the previous claims, wherein at least one classifying descriptor is binning the respective value into one of not more than five ordered classes, in particular into not more than four ordered classes, in particular preferably into one of three ordered classes,
and wherein preferably all classifying descriptors are binning the respective value into one of not more than five classes, in particular into not more than four classes, in particular preferably into one of three classes.

8. The method according to one of the previous claims,
wherein the maximum possible contribution to the combined score of the descriptor relating to indicating whether or not the neoantigen is known to be cancer-related is larger than the maximum possible contribution to the combined score of any single of the descriptors relating to a relative expression rate in one or more cancerous cells of the subject, a binding affinity to particular HLA alleles present according to the subject's HLA type, a relative HLA binding affinity of the subject specific potential neoantigen as compared to the corresponding non-mutated wild-type sequence, a binding affinity to more than one HLA allele present according to the subject's HLA type, an HLA promiscuity and the reliability of predicting binding of the subject specific potential neoantigen; and/or
wherein the maximum possible contribution to the combined score of the descriptor relating to a relative expression rate in one or more cancerous cells of the subject is larger than the maximum possible contribution to the combined score of any single of the descriptors relating toa binding affinity to particular HLA alleles present according to the subject's HLA type, a relative HLA binding affinity of the subject specific potential neoantigen as compared to the corresponding non-mutated wild-type sequence, a binding affinity to more than one HLA allele present according to the subject's HLA type, an HLA promiscuity, and the reliability of predicting binding of the subject specific potential neoantigen;
and/or
wherein the maximum possible contribution to the combined score of the descriptor relating to a binding affinity to particular HLA alleles present according to the subject's HLA type is larger than the maximum possible contribution to the combined score of any single of the descriptors relating to a relative HLA binding affinity of the subject specific potential neoantigen as compared to the corresponding non-mutated wild-type sequence, a binding affinity to more than one HLA allele present according to the subject's HLA type,an HLA promiscuity, and the reliability of predicting binding of the subject specific potential neoantigen; and/or
wherein the maximum possible contribution to the combined score of the descriptor relating to a relative HLA binding affinity of the subject specific potential neoantigen as compared to the corresponding non-mutated wild-type sequence is larger than the maximum possible contribution to the combined score of any single of the descriptors relating to a binding affinity to more than one HLA allele present according to the subject's HLA type, an HLA promiscuity, and the reliability of predicting binding of the subject specific potential neoantigen; and/or
wherein the maximum possible contribution to the combined score of the descriptor relating to a binding affinity to more than one HLA allele present according to the subject's HLA type is larger than the maximum possible contribution to the combined score of any single of the descriptors relating to an HLA promiscuity and the reliability of predicting binding of the subject specific potential neoantigen; and/or the maximum possible contribution to the combined score of the descriptor relating to an HLA promiscuity is larger than the maximum possible contribution to the combined score of the descriptors relating to the reliability of predicting binding of the subject specific potential neoantigen,
in particular a method where each of the respective possible contributions to the combined score mentioned above obeys the relations indicated.

9. The method according to any of the previous claims, wherein a classifying descriptor relating to the binning of a value indicative for an allele frequency of the at least one tumor-specific mutation in the neoantigen of the subject into one of at three different classes ordered according to the intervals of values binned into each class is determined such that a tumor content Y is defined and the value of the allele frequency is defined to be in the highest class if the allele frequency is at least 1/3 of the tumor content, to be in the lowest class if the allele frequency is no more than 1/6 of the tumor content Y and else to be in the medium class, and the maximum contribution of the corresponding classifying descriptor if the allele frequency is in the medium class being less than the contribution in case of a highest class and the contribution in case of a lowest class.

10. A selection method for cancer-specific neoantigen selection wherein a ranking according to the previous claim is determined and at least one neoantigen, preferably at least two neoantigens, and less than all neoantigens from the plurality of potential neoantigens in view of the ranking is selected,
in particular
a selection method, wherein an ensemble consisting of a plurality of different neoantigens is selected, the neoantigens of the ensemble being selected in view of their ranking such that for each of a plurality of the HLA alleles considered, the nonfiltered most favorable ranked neoantigen is selected, preferably for each HLA allele the nonfiltered most favorable ranked neoantigen is selected,
and such that, if the ensemble comprises more neoantigens than these most favorably ranked neoantigens, then further neoantigens for different alleles are selected starting with HLA-A or B alleles; and preferably further such that if at least two such neoantigens for the same variant, but different alleles starting with HLA-A or B alleles are equally ranked, then a neoantigen with an HLA type hitherto underrepresented in the ensemble is selected, else if at least two such neoantigens for a different variant, but same HLA are equally ranked, then the neoantigen having the higher expression is selected; and preferably further such that both for the case where neoantigens are selected according to their higher expression or the case where a neoantigen with an HLA type hitherto underrepresented in the ensemble is selected, if at least two such neoantigens are equally ranked, then a neoantigen thereof with a higher affinity is selected, preferably a higher affinity according to not the classifying descriptor but according to the original value classified, and preferably further such that if at least two such neoantigens having an equal affinity exist, then the neoantigen having a higher promiscuity is selected and preferably further such that if at least two such neoantigens having an equal affinity exist, then the neoantigen having a lower hydrophobicity is selected,
in particular a method wherein an ensemble of at least 3 neoantigens is selected.

11. A method according to one of the previous claims,
wherein HLA alleles are considered to be subject to a HLA haplotype reduction derived in view of a tumor transcriptome, a tumor exome or a blood exome or an immunohistochemistry staining of a tumor tissue sample;
and/or wherein
the method is for selecting at least one each of HLA class I restricted neoantigens and HLA class II restricted neoantigens.

12. A pharmaceutical composition
comprising at least one substance determined in response to a result of a selection method according to one of claims 1-11, and wherein in particular the pharmaceutical composition is a pharmaceutical composition for use in treating cancer
and/or
comprising a patient-specific cocktail of neoantigen or neoepitope peptides determined by a method accordingly to one of claim 1 - 11, and a pharmaceutically acceptable excipient, in particular for use in treating cancer.

13. Use of a neoantigen selected in accordance with a method accordingly to any of claims 1 - 11 in preparing a personalized pharmaceutical composition.

14. A data carrier comprising data relatable to at least one individual patient having cancer, the data carrier carrying data relating to a plurality of potential neoantigens carrying at least one mutation considered to be specific to the cancer of the at least one individual patient in that for each at least four potential antigens of this plurality of neoantigens at least two of the group (a) thru (h) are provided, with the group (a) thru (h) consisting of
(a) an indicative descriptor indicating whether the neoantigen is known to reside within a cancer-related gene or whether the neoantigen is not known to reside within a cancer-related gene
and/or a value indicative for a likelihood estimate the neoantigen has to be not cancer-related;
(b) a classifying descriptor relating to the binning of a value indicative for an allele frequency of the at least one tumor-specific mutation in the neoantigen of the subject into one of at least three different classes ordered according to the intervals of values binned into each class
and/or a value indicative for an allele frequency of the at least one tumor-specific mutation in the neoantigen of the subject;
(c) a classifying descriptor relating to the binning of a value indicative for a relative expression rate of the at least one variant within a neoantigen in one or more cancerous cells of the subject into one of at least three different classes ordered according to the intervals of values binned into each class
and/or a value indicative for a relative expression rate of the at least one variant within a neoantigen in one or more cancerous cells of the subject;
(d) a classifying descriptor relating to the binning of a value indicative for a binding affinity of a neoantigen to particular HLA alleles present according to the subject's HLA type, into one of at least two different classes ordered according to the intervals of values binned into each class
and/or a value indicative for a binding affinity of a neoantigen to particular HLA alleles present according to the subject's HLA type;
(e) a classifying descriptor relating to the binning of a value indicative for a relative HLA binding affinity of the subject specific potential neoantigen as compared to the corresponding non-mutated wild-type sequence into one of at least three different classes ordered according to the intervals of values binned into each class
and/or a value indicative for a relative HLA binding affinity of the subject specific potential neoantigen as compared to the corresponding non-mutated wild-type sequence;
(f) a classifying descriptor relating to the binning of a value indicative for a binding affinity to more than one HLA allele present according to the subject's HLA type, into one of at least three different classes ordered according to the intervals of values binned into each class
and/or a value indicative for a binding affinity to more than one HLA allele present according to the subject's HLA type;
(g) a classifying descriptor relating to the binning of a value indicative for the HLA promiscuity of a neoantigen into one of at least three different classes ordered according to the intervals of values binned into each class
and/or a value indicative for the HLA promiscuity of a neoantigen;
(h) a classifying descriptor relating to the binning of a value indicative for the reliability of predicting binding of the subject specific potential neoantigen to a HLA allele of the respective patient into one of at least three different classes ordered according to the intervals of values binned into each class and/or a value indicative for the reliability of predicting binding of the subject specific potential neoantigen to a HLA allele of the respective patient;
and/or the data carrier carrying data carrying data relating to neoantigens scoring as obtained by one of the previously claimed methods;
and/or the data carrier carrying data relating or one or more neoantigens selected according to one of the preceding claims;
and/or the data carrier carrying data relating to instructions to produce a pharmaceutical composition comprising at least one substance determined in response to a result of a selection method according to one of the preceding method claims.

15. A kit comprising at least one of a container for biological material prepared in a manner allowing determination of personalized data usable as input into a method according to one of claims 1 - 11 wherein said biological material is obtained from a patient having cancer; or a data carrier storing personalized genetic data usable as individual-related input into said method and an information carrier carrying information relating to the identification of the patient; and instructions to execute said method and/or to provide data for the production of a data carrier as defined in the previous claim and/or to provide a data carrier.
